# EUROPEAN PATENT SPECIFICATION

(11) **EP 3 134 738 B1**
(45) Date of publication and mention of the grant of the patent: **24.07.2019**
(21) Application number: 15782624.9
(22) Date of filing: 25.04.2015
(51) Int. Cl.: G01N 35/08, C12M 1/00, C12M 1/34

(54) **DEP FORCE CONTROL AND ELECTROWETTING CONTROL IN DIFFERENT SECTIONS OF THE SAME MICROFLUIDIC APPARATUS**
DEP-KRAFTSTEUERUNG UND ELEKTROBENETZUNGSSTEUERUNG IN VERSCHIEDENEN ABSCHNITTEN EINER MIKROFLUIDISCHEN VORRICHTUNG
COMMANDE DE FORCE DEP ET COMMANDE D'ÉLECTROMOUILLAGE DANS DIFFÉRENTES SECTIONS DU MÊME APPAREIL MICROFLUIDIQUE

(30) Priority: 25.04.2014 US 201414262140
(43) Date of publication of application: 01.03.2017
(73) Proprietor: Berkeley Lights, Inc., Emeryville, CA 94608 (US)
(72) Inventor: KHANDROS, Igor Y., Emeryville, California 94608 (US); NEVILL, J. Tanner, Emeryville, California 94608 (US); SHORT, Steven W., Emeryville, California 94608 (US); WU, Ming C., Emeryville, California 94608 (US)
(74) Representative: D Young & Co LLP
(86) International application number: PCT/US2015/027679
(87) International publication number: WO 2015/164846

(56) References cited:
- WO-A2-2009/130694
- KR-A- 20120 066 100
- US-A1- 2003 224 528
- US-A1- 2007 023 292
- US-A1- 2008 038 810
- US-A1- 2010 101 960
- US-A1- 2010 181 195
- US-A1- 2011 086 377
- US-A1- 2012 024 708

## Description

### CROSS REFERENCE TO RELATED APPLICATION(S)

This application claims priority to U.S. Patent Application No. 14/262,140, filed April 25, 2014.

### BACKGROUND

Micro-objects, such as biological cells, can be processed in microfluidic apparatuses. For example, micro-objects suspended in a liquid in a microfluidic apparatus can be sorted, selected, and moved in the microfluidic apparatus. The liquid can also be manipulated in the device. Embodiments of the present invention are directed to improvements in selectively generating net DEP forces in a first section of a microfluidic apparatus and changing an effective wetting property of an electrowetting surface in another section of the microfluidic apparatus.
KR 2012 0066100 A discloses a device for blood analysis comprising a transparent substrate, a middle part, and a lower substrate. The transparent substrate has a discharge hole and is coated with hydrophobic dielectric layer. The middle part has an inlet reservoir part and outlet reservoir part. An electrode has an electrode for dielectrophoresis and an electrode for electrowetting.
WO2009130694 A2 discloses cell carrier structures using dynamic flow of cell bearing, washing, or nourishing fluid, from an input reservoir region to an output reservoir region. A common feature of the structures is the presence of channels generally having low height or other cross section, such that the cell bearing fluid traverses these channels by capillary action.

### SUMMARY

According to a first aspect of the invention, there is provided an apparatus as defined in claim 1.

According to a second aspect of the invention, there is provided a process of operating a fluidic apparatus, the process as defined in claim 9.

The apparatus includes an enclosure, a dielectrophoresis (DEP) configuration, and an electrowetting (EW) configuration. The enclosure comprises a first surface and an electrowetting surface. The DEP configuration is configured to selectively induce net DEP forces in a first liquid medium disposed on the first surface, and the EW
configuration is configured to selectively change an effective wetting property of the electrowetting surface.

In some embodiments, the process of operating a fluidic apparatus can include inducing a net DEP force on a micro-object in a first liquid medium on a first surface in a first section of the apparatus. The process can also include changing an effective wetting property of a region of an electrowetting surface on which a second liquid medium is disposed in a second section of the apparatus.

The apparatus comprises an enclosure and a boundary. The enclosure is configured to hold a first liquid medium disposed on a first surface in a first section of the enclosure and a second liquid medium disposed on an electrowetting surface in a second section of the enclosure. The boundary is between the first section and the second section of the enclosure. The first section of the enclosure comprises a DEP configuration configured to induce selectively net DEP forces in the first liquid medium sufficiently to capture
and move, relative to the first surface, micro-objects in the first liquid medium in the first section of the enclosure, while the first section is connected to a biasing device. The second section of the enclosure comprises an EW configuration configured to change selectively an effective wetting characteristic of regions of the electrowetting surface sufficiently to move a liquid droplet within the second medium in the second section of the enclosure, while the second section is connected to a biasing device.

The process of operating a fluidic apparatus includes drawing a droplet of a first liquid medium disposed on a first surface in a first section of an enclosure into a second medium disposed on an electrowetting surface in a second section of the enclosure. The foregoing drawing includes changing an effective electrowetting characteristic of a region of the electrowetting surface at a boundary with the first surface and thereby induce a force at the boundary that is sufficient to draw a droplet across the boundary and into the second liquid medium.

### BRIEF DESCRIPTION OF THE DRAWINGS

Figure 1A is a perspective view of a microfluidic apparatus comprising sections for holding different liquid medium, inducing net dielectrophoresis (DEP) forces in one section and controlling an effective electrowetting property of a surface of another of the sections according to some embodiments of the invention.
Figure 1B is a cross-sectional side view of the microfluidic apparatus of Figure 1A.
Figure 1C is a top view of the microfluidic apparatus of Figure 1A with the cover removed.
Figure 2 is a cross-sectional side view of the micro-fluidic device of Figure 1A with liquid media in its sections and connected to biasing devices according to some embodiments of the invention.
Figure 3 illustrates an example of a DEP configuration and a controllable electrowetting (EW) configuration of the enclosure of the device of Figure 1A according to some embodiments of the invention.
Figure 4 is an example of the electrode activation substrate of Figure 3 configured as photoconductive material according to some embodiments of the invention.
Figure 5 is another example of the electrode activation substrate of Figure 3 configured as a circuit substrate according to some embodiments of the invention.
Figure 6 illustrates another example of a DEP configuration and an EW configuration of the enclosure of the device of Figure 1A according to some embodiments of the invention.
Figure 7 is yet another example of a DEP configuration and an EW configuration of the enclosure of the device of Figure 1A according to some embodiments of the invention.
Figure 8 is a cross-sectional side view of a microfluidic apparatus with multiple stacked sections according to some embodiments of the invention.
Figure 9 illustrates another example of an embodiment of a microfluidic apparatus with multiple stacked sections according to some embodiments of the invention.
Figure 10A is a perspective view of an example of a microfluidic apparatus comprising a DEP configuration for manipulating micro-objects in a first section of the device and an EW configuration for manipulating droplets of a liquid medium on an electrowetting surface in a second section of the device according to some embodiments of the invention.
Figure 10B is a side cross-sectional view of the microfluidic apparatus of Figure 10A.
Figure 10C is a top view of the microfluidic apparatus of Figure 10A with the cover removed.
Figure 11 is an example of a process for moving a micro-object from a first liquid medium in a first section of a microfluidic apparatus into a second liquid medium in a second section of the microfluidic apparatus according to some embodiments of the invention.
Figures 12A-21 show examples of performance of the process of Figure 11 according to some embodiments of the invention.
Figure 22 is an example of a process for culturing biological micro-objects in a microfluidic apparatus configured to hold multiple different liquid media according to some embodiments of the invention.
Figures 23-26 illustrate an example of performance of the process of Figure 22 according to some embodiments of the invention.
Figure 27 shows an example of a process that can be performed on the microfluidic apparatus of Figures 1A-1C or the microfluidic apparatus of Figures 10A-10C according to some embodiments of the invention.
Figure 28 illustrates an example in which a droplet generator is used to produce droplets in a channel of a microfluidic circuit according to some embodiments of the invention.
Figures 29 and 30 show variations of the microfluidic circuit of Figure 28.
Figure 31 is an example of a process for analyzing biological micro-objects in the microfluidic circuits of Figures 28-30.

### DETAILED DESCRIPTION OF EXEMPLARY EMBODIMENTS

This specification describes exemplary embodiments and applications of the invention. The invention, however, is not limited to these exemplary embodiments and applications or to the manner in which the exemplary embodiments and applications operate or are described herein. Moreover, the figures may show simplified or partial views, and the dimensions of elements in the figures may be exaggerated or otherwise not in proportion. In addition, as the terms "on," "attached to," or "coupled to" are used herein, one element (e.g., a material, a layer, a substrate, etc.) can be "on," "attached to," or "coupled to" another element regardless of whether the one element is directly on, attached to, or coupled to the other element or there are one or more intervening elements between the one element and the other element. Also, directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. In addition, where reference is made to a list of elements (e.g., elements a, b, c), such reference is intended to include any one of the listed elements by itself, any combination of less than all of the listed elements, and/or a combination of all of the listed elements.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent. The term "ones" means more than one.

As used herein, the term "micro-object" can encompass one or more of the following: inanimate micro-objects such as microparticles, microbeads (e.g., polystyrene beads, Luminex™ beads, or the like), magnetic or paramagnetic beads (e.g. solid phase reversible immobilization (SPRI) beads), microrods, microwires, quantum dots, and the like; biological micro-objects such as cells (e.g., embryos, oocytes, sperms, cells dissociated from a tissue, blood cells, hybridomas, cultured cells, cells from a cell line, cancer cells, infected cells, transfected and/or transformed cells, reporter cells, and the like), liposomes (e.g., synthetic or derived from membrane preparations), lipid nanorafts, and the like; or a combination of inanimate micro-objects and biological micro-objects (e.g., microbeads attached to cells, liposome-coated micro-beads, liposome-coated magnetic beads, or the like). Lipid nanorafts have been described, e.g., in Ritchie et al. (2009) "Reconstitution of Membrane Proteins in Phospholipid Bilayer Nanodiscs," Methods Enzymol., 464:211-231.

As used herein, the term "cell" refers to a biological cell, which can be a plant cell, an animal cell (e.g., a mammalian cell), a bacterial cell, a fungal cell, or the like. A mammalian cell can be, for example, from a human, a mouse, a rat, a horse, a goat, a sheep, a cow, a primate, or the like, and can include any of the following cell types: oocytes, sperm, embryos, blood cells, immunological cells, macrophages, NK cells, T cells, B cells, hybridomas, cancer cells, stem cells, normal cells, infected cells (e.g., infected with a virus or other parasite), cells dissociated from a tissue, cultured cells, cells from a cell line, transfected and/or transformed cells, reporter cells, and the like.

A colony of biological cells is "clonal" if all of the living cells in the colony that are capable of reproducing are daughter cells derived from a single parent cell. The term "clonal cells" refers to cells of the same clonal colony.

The phrase "relatively high electrical conductivity" is used herein synonymously with the phrase "relatively low electrical impedance," and the foregoing phrases are interchangeable. Similarly, the phrase "relatively low electrical conductivity" is used synonymously with the phrase "relatively high electrical impedance," and the foregoing phrases are interchangeable.

A "fluidic circuit" means one or more fluidic structures (e.g., chambers, channels, holding pens, reservoirs, or the like), which can be interconnected. A "fluidic circuit frame" means one or more walls that define all or part of a fluidic circuit. A "holding pen" means a region in a microfluidic device, defined by walls of the fluidic circuit frame and having at least one opening to a different region of the microfluidic device (e.g., a channel, chamber, or another holding pen), which is configured to hold a volume of fluid and, optionally, one or more micro-objects. A holding pen can be an isolation chamber that contains an isolation region (e.g., an unswept region, as discussed below).

As used herein, the term "maintaining (a) cell(s)" refers to providing an environment comprising both fluidic and gaseous components and, optionally a surface, that provides the conditions necessary to keep the cells viable and/or expanding.

A "component" of a fluidic medium is any chemical or biochemical molecule present in the medium, including solvent molecules, ions, small molecules, antibiotics, nucleotides and nucleosides, nucleic acids, amino acids, peptides, proteins, sugars, carbohydrates, lipids, fatty acids, cholesterol, metabolites, or the like.

As used herein in reference to a fluidic medium, "diffuse" and "diffusion" refer to thermodynamic movement of a component of the fluidic medium down a concentration gradient.

The phrase "flow of a medium" means bulk movement of a fluidic medium primarily due to any mechanism other than diffusion. For example, flow of a medium can involve movement of the fluidic medium from one point to another point due to a pressure differential between the points. Such flow can include a continuous, pulsed, periodic, random, intermittent, or reciprocating flow of the liquid, or any combination thereof. When one fluidic medium flows into another fluidic medium, turbulence and mixing of the media can result.

The phrase "substantially no flow" refers to a rate of flow of a fluidic medium that, averaged over time, is less than the rate of diffusion of components of a material (e.g., an analyte of interest) into or within the fluidic medium. The rate of diffusion of components of such a material can depend on, for example, temperature, the size of the components, and the strength of interactions between the components and the fluidic medium.

As used herein in reference to different regions within a microfluidic device, the phrase "fluidically connected" means that, when the different regions are substantially filled with fluid, such as fluidic media, the fluid in each of the regions is connected so as to form a single body of fluid. This does not mean that the fluids (or fluidic media) in the different regions are necessarily identical in composition. Rather, the fluids in different fluidically connected regions of a microfluidic device can have different compositions (e.g., different concentrations of solutes, such as proteins, carbohydrates, ions, or other molecules) which are in flux as solutes move down their respective concentration gradients and/or fluids flow through the device.

In some embodiments, a microfluidic device can comprise "swept" regions and "unswept" regions. A swept region is a region in which fluid is able to flow, whereas an unswept region is a region in which (given the configuration of the microfluidic device) fluid generally is unable to flow. An unswept region can be fluidically connected to a swept region, provided the fluidic connections are structured to enable diffusion but substantially no flow of media between the swept region and the unswept region. The microfluidic device can thus be structured to substantially isolate an unswept region from a flow of medium in a swept region, while enabling substantially only diffusive fluidic communication between the swept region and the unswept region. Microfluidic devices having swept and unswept regions have been described, for example, in U.S. Patent Application No. 14/520,568, filed October 22, 2014.

A "microfluidic channel" or "flow channel" as used herein refers to flow region (or swept region) of a microfluidic device having a length that is significantly longer than both the horizontal and vertical dimensions. For example, the flow channel can be at least 5 times the length of either the horizontal or vertical dimension, e.g., at least 10 times the length, at least 25 times the length, at least 100 times the length, at least 200 times the length, at least 500 times the length, at least 1,000 times the length, at least 5,000 times the length, or longer. In some embodiments, the length of a flow channel is in the range of from about 100,000 microns to about 500,000 microns, including any range therebetween. In some embodiments, the horizontal dimension is in the range of from about 100 microns to about 300 microns (e.g., about 200 microns) and the vertical dimension is in the range of from about 25 microns to about 100 microns, e.g., from about 40 to about 50 microns. It is noted that a flow channel may have a variety of different spatial configurations in a microfluidic device, and thus is not restricted to a perfectly linear element. For example, a flow channel may be, or include one or more sections having, the following configurations: curve, bend, spiral, incline, decline, fork (e.g., multiple different flow paths), and any combination thereof. In addition, a flow channel may have different cross-sectional areas along its path, widening and constricting to provide a desired fluid flow therein.

The capability of biological micro-objects (e.g., biological cells) to produce specific biological materials (e.g., proteins, such as antibodies) can be assayed in a microfluidic device having a swept region, such as a channel, and an unswept region, such as an isolation pen (or isolation chamber). For example, sample material comprising biological micro-objects (e.g., cells) to be assayed for production of an analyte of interest can be loaded into a swept region of the microfluidic device. Ones of the biological micro-objects (e.g., mammalian cells, such as human cells) can be selected for particular characteristics and disposed in unswept regions. The remaining sample material can then be flowed out of the swept region and an assay material flowed into the swept region. Because the selected biological micro-objects are in unswept regions, the selected biological micro-objects are not substantially affected by the flowing out of the remaining sample material or the flowing in of the assay material. The selected biological micro-objects can be allowed to produce the analyte of interest, which can diffuse from the unswept regions into the swept region, where the analyte of interest can react with the assay material to produce localized detectable reactions, each of which can be correlated to a particular unswept region. Any unswept region associated with a detected reaction can be analyzed to determine which, if any, of the biological micro-objects in the unswept region are sufficient producers of the analyte of interest.

Similarly, biological micro-objects, such as cells, can be cultured or grown, after being placed in an unswept region (e.g., isolation region of an isolation chamber), by flowing culture medium though a swept region (e.g., a flow channel) to which the unswept region is fluidically connected. As the biological micro-objects are being cultured, nutrients from the culture medium in the swept region will diffuse into the unswept region, where they can be absorbed and used by the biological micro-objects, while waste products produced by the biological micro-objects and released into the unswept region can diffuse out of the unswept region and into the swept region, at which point the waste products can be flowed away (e.g., out of the microfluidic device).

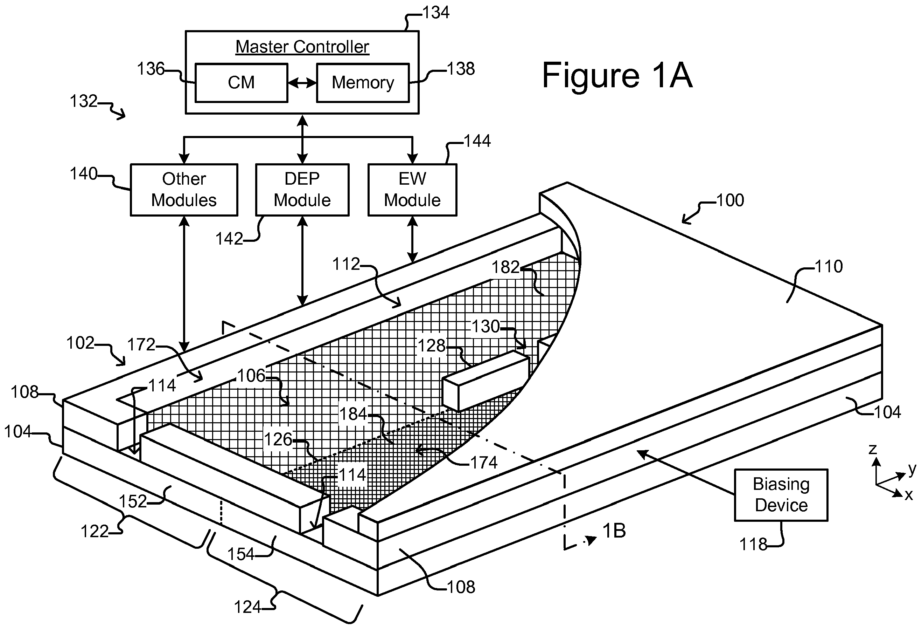

In some embodiments, a microfluidic apparatus can comprise a dielectrophoresis (DEP) configured section for holding a liquid medium and selectively inducing net DEP forces in the liquid medium. The microfluidic apparatus can also comprise an electrowetting (EW) configured section for holding another liquid medium in contact with an electrowetting surface and selectively changing an effective wetting property of the electrowetting surface. Figures 1A-1C illustrate an example of such a microfluidic apparatus 100. Figure 1A also illustrates examples of control equipment 132 for controlling operation of the apparatus 100.

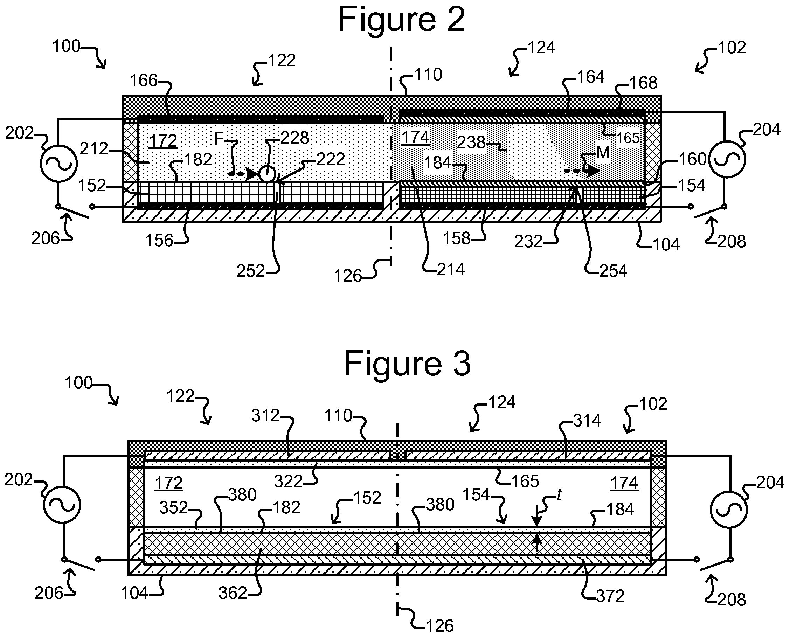

As shown, the apparatus 100 can comprise an enclosure 102, which can comprise a plurality (two are shown but there can be more) of sections 122, 124 each configured to hold a liquid medium (not shown in Figures 1A-1C, but depicted as 212, 214 in Figure 2). The first section 122 can comprise a first surface 182 and be further configured to selectively generate net DEP forces on micro-objects (not shown) in a liquid medium in contact with the first surface 182. The first section 122 is thus referred to hereinafter as a DEP configured section or a DEP configuration 122 of the enclosure 102. The second section 124 can comprise an electrowetting surface 184 and can further be configured to selectively change an effective wetting property of the electrowetting surface 184. The second section 124 is thus referred to hereinafter as an electrowetting (EW) configured section or an EW configuration 124 of the enclosure 102.

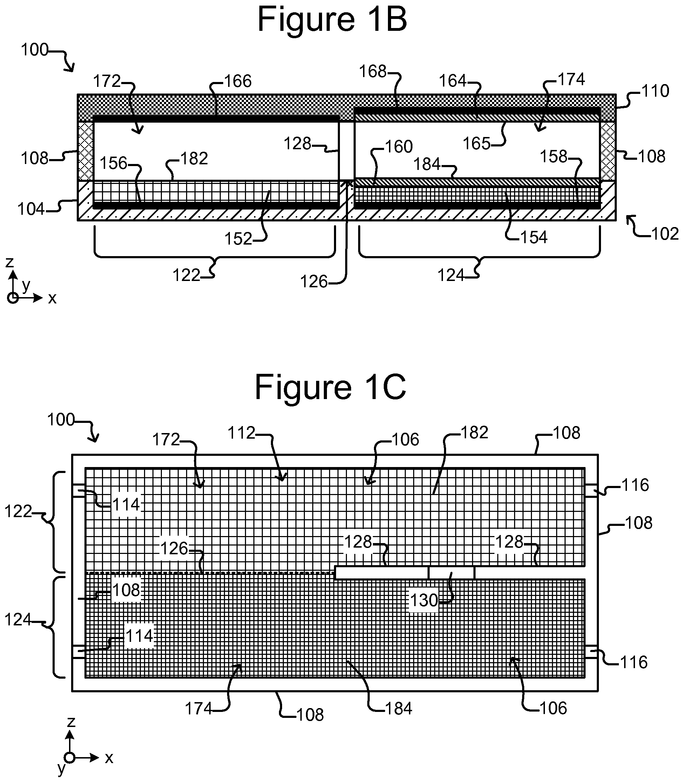

Although the apparatus 100 can be physically structured in many different ways, in the example shown in Figures 1A-1C, the enclosure 102 is depicted as comprising a structure 104 (e.g., a base), a fluidic circuit frame 108, and a cover 110. As shown, the fluidic circuit frame 108 can be disposed on an inner surface 106 of the structure 104, and the cover 110 can be disposed over the fluidic circuit frame 108. With the structure 104 as the bottom and the cover as the top 110, the fluidic circuit frame 108 can define a fluidic circuit comprising, for example, interconnected fluidic chambers, channels, pens, reservoirs, and the like. Although the structure 104 is shown in Figures 1A and 1B as comprising the bottom of the apparatus 100 and the cover 110 is illustrated as the top, the structure 104 can be the top and the cover 110 can be the bottom of the apparatus 100.

In the example illustrated in Figures 1A-1C, the fluidic circuit frame 108 defines a chamber 112. A first section 172 of the chamber 112 corresponding to a DEP configured section 122 is hereinafter referred to as the first chamber section 172, and a second section of the chamber 112 corresponding to an EW section 124 of the enclosure 102 is hereinafter referred to as the second chamber section 174. As also shown, the chamber 112 can include one or more inlets 114 and one or more outlets 116.

In some embodiments, the enclosure 102 can comprise a physical barrier 128 between the first chamber section 172 and the second chamber section 174, and such a physical barrier 128 can comprise one or more passages 130 from the first chamber section 172 of the enclosure 102 to the second chamber section 174. In the example illustrated in Figures 1A-1C, such a physical barrier 128 is shown along only a portion of a boundary 126 between the first chamber section 172 and the second chamber section 174. Alternatively, the physical barrier 128 can extend the entirety of the boundary 126 or be located on a different portion of the boundary 126. Regardless, the physical barrier 128 can be part of the fluidic circuit frame 108 (as shown), or the physical barrier 128 can be structurally distinct from the fluidic circuit frame 108. Although one physical barrier 128 is shown, there can be more than one such physical barrier 128 disposed on the boundary 126.

The structure 104 can comprise, for example, a substrate (e.g., a photoconductive substrate or a circuit substrate) or a plurality of such substrates that are interconnected. The fluidic circuit frame 108 can comprise a material, which can be flexible or gas permeable. Alternatively, the material need not be flexible and/or gas permeable. Suitable examples of materials that the circuit frame 108 can comprise include rubber, plastic, an elastomer, silicone, photo-patternable silicon (PPS), polydimethylsiloxane ("PDMS "), or the like. The cover 110 can be an integral part of the fluidic circuit frame 108, or the cover 110 can be a structurally distinct element (as illustrated in Figures 1A-1C). The cover 110 can comprise the same materials as the fluidic circuit frame 108. Thus, the cover 110 can be made from or comprise a flexible material, as discussed above. Alternatively, the cover 110 can be made from or comprise a rigid material (e.g., glass, including ITO-coated glass). Regardless, the cover 110 and/or the structure 104 can be transparent to light.

As shown in Figure 1B, in some embodiments, the DEP configuration 122 of the enclosure 102 can comprise a biasing electrode 156, a DEP section 152 of the structure 104, and the first surface 182, all of which can be part of the structure 104. The DEP configuration 122 can also include a biasing electrode 166, which can be part of the cover 110. The foregoing can be located with respect to each other as illustrated in Figure 1B. The first surface 182 can be an outer surface of the DEP section 152 or an outer surface of one or more materials (e.g., one or more coatings) (not shown) disposed on the DEP section 152. Examples of such coatings (not shown) on the first surface 182 include electrically insulating materials.

Similarly, the EW configuration 124 of the enclosure 102 can comprise a biasing electrode 158, an EW section 154 of the structure 104, a dielectric layer 160, and the electrowetting surface 184, all of which can be part of the structure 104. The EW configuration 124 can also include a hydrophobic surface 165, a layer 164 (e.g., a dielectric material), and a biasing electrode 168, all of which can be part of the cover 110. The foregoing can be located with respect to each other as shown in Figure 1B. The dielectric layer 160 and/or the layer 164 can comprise a hydrophilic material such as silicon oxide (SiO₂), aluminum oxide (Al₃O₂), or the like. Alternatively, the dielectric layer 160 and/or the layer 164 can comprise a hydrophobic material such as a hydrophobic polymer (e.g., a perfluoro-polymer, such as CYTOP, or a poly(p-xylylen)polymer, such as parylene). The electrowetting surface 184, which can be hydrophobic, can be an outer surface of the dielectric layer 160 or an outer surface of one or more materials (not shown) disposed on the dielectric layer 160. Similarly, the hydrophobic surface 165 can be an outer surface of the layer 164 or an outer surface of one or more materials (not shown) disposed on the layer 164. An example of a material that can be disposed on the dielectric layer 160 and/or the layer 164 includes polytetrafluoroethylene (PTFE, a.k.a. Teflon™ by Dupont™).

As shown in Figure 1A, an electrical biasing device 118 can be connected to the apparatus 100. The electrical biasing device 118 can, for example, comprise one or more voltage or current sources. As also shown in Figure 1A, examples of the control equipment include a master controller 134, a DEP module 142 for controlling the DEP configuration 122 of the enclosure 102, and an EW module 144 for controlling the EW configuration 124 of the enclosure 102. The control equipment 132 can also include other modules 140 for controlling, monitoring, or performing other functions with respect to the apparatus 100.

The master controller 134 can comprise a control module 136 and a digital memory 138. The control module 136 can comprise, for example, a digital processor configured to operate in accordance with machine executable instructions (e.g., software, firmware, microcode, or the like) stored in the memory 138. Alternatively or in addition, the control module 136 can comprise hardwired digital circuitry and/or analog circuitry. The DEP module 142, EW module 144, and/or the other modules 140 can be similarly configured. Thus, functions, processes, acts, actions, or steps of a process discussed herein as being performed with respect to the apparatus 100 or any other microfluidic apparatus can be performed by one or more of the master controller 134, DEP module 142, EW module 144, or other modules 140 configured as discussed above.

Figure 2 illustrates an example configuration of the apparatus 100. As shown, a first liquid medium 212 can be disposed on the first surface 182 in the first chamber section 172, and a second liquid medium 214 can be disposed on the electrowetting surface 184 in the second chamber section 174. The first liquid medium 212 and the second liquid medium 214 can be different mediums. For example, the second liquid medium 214 can be immiscible with respect to the first liquid medium 212. The first liquid medium 212 can be, for example, an aqueous medium, such as water, an aqueous buffer (e.g., a phosphate buffer, a tris(hydroxymethyl)amionmethane (Tris) buffer, or the like), an aqueous solution (e.g., containing one or more soluble active agents), cell culture medium, etc. The second liquid medium 214 can be immiscible in an aqueous medium. Examples of the second liquid medium 214 can include oil based media. Examples of suitable oils include gas permeable oils such as fluorinated oils. Fluorocarbon based oils are also examples of suitable oils.

As also shown in Figure 2, a first biasing device 202 can be connected to the biasing electrodes 156, 166 of the DEP configuration 122 of the enclosure 102, and a second biasing device 204 can be connected to the biasing electrodes 158, 168 of the EW configuration 124 of the enclosure 102. The first biasing device 202 can be, for example, an alternating current (AC) voltage or current source, and the second biasing device 204 can similarly be an AC voltage or current source. A switch 206 can selectively connect the first biasing device 202 to and disconnect the first biasing device 202 from the DEP configuration 122. Another switch 208 can similarly connect the second biasing device 204 to and disconnect the second biasing device 204 from the EW configuration 124. The biasing devices 202, 204 and switches 206, 208 can be part of the biasing device 118 of Figure 1A.

The DEP section 152 of the structure 104 can be configured to have a relatively high electrical impedance (i.e., a relatively low electrical conductivity) between the first medium 212 and the biasing electrode 156 except when a DEP electrode 222 at the first surface 182 is activated. (The DEP section 152 can be an example of an electrode activation substrate.) Activating the DEP electrode 222 can create a relatively low electrical impedance (i.e., a relatively high electrical conductivity) path 252 from the DEP electrode 222 to the biasing electrode 156. While the DEP electrode 222 is deactivated, the majority of the voltage drop due to the first biasing device 202 from the DEP biasing electrode 166 to the DEP biasing electrode 156 can be across the DEP section 152. While the DEP electrode 222 is activated and creating the relatively low electrical impedance path 252, however, the majority of the voltage drop in the vicinity of the path 252 can be across the first medium 222, which can create a net DEP force (F) in the first medium 212 in the vicinity of the activated DEP electrode 222. Depending on such characteristics as the frequency of the biasing device 202 and the dielectric properties of the first medium 212 and/or micro-objects 228 in the medium 212, the DEP force F can attract or repel a nearby micro-object 228 in the first medium 212. Many DEP electrodes like DEP electrode 222 can be selectively activated and deactivated over some, most, or the entirety of the first surface 182. By selectively activating and deactivating such DEP electrodes (like 222), one or more micro-objects 228 in the first medium 212 of the DEP section 152 of the enclosure 102 can be selected (e.g., captured) and moved (e.g., in a directed manner) in the medium 212. Equipment 132 (see Figure 1A) can control activation and deactivation of such DEP electrodes (e.g., 222). As will be seen, DEP electrodes (like 222) can be fixed in a particular location, in the manner of conventional electrodes (e.g., metal electrodes), phototransistors, or photo-actuated electrodes. Alternatively, DEP electrodes (like 222) can be virtual electrodes that are located at positions where electromagnetic radiation is incident on a photoconductive material, as occurs when light of an appropriate frequency is incident on a layer of amorphous silicon that is connected to a biasing electrode (like 156).

The EW section 154 of the structure 104 can similarly be configured to have a relatively high electrical impedance (i.e., relatively low electrical conductivity) except when an EW electrode 232 at the electrowetting surface 184 is activated. (The EW section 154 can also be an example of an electrode activation substrate.) Activating such an EW electrode 232 can create a relatively low electrical impedance (i.e., a relatively high electrical conductivity) path 254 from the EW electrode 232 to the EW biasing electrode 158. While the EW electrode 232 is deactivated (and the EW section 154 has a relatively high electrical impedance), the voltage drop due to the second biasing device 204 from the EW biasing electrode 168 to the EW biasing electrode 158 can be greater across the EW section 154 than across the dielectric layer 160. While the EW electrode 232 is activated and creating the relatively low electrical impedance path 254, however, the voltage drop across the EW section 154 can become less than the voltage drop across the dielectric layer 160.

The foregoing can change a force across the EW surface 184, which can change an effective wetting property of the EW surface 184 in the vicinity of the activated EW electrode 232. For example, as noted, the EW surface 184 can be hydrophobic. Activating an EW electrode 232 can increase a Coulombic force across the EW surface 184 (due to increased charge density at the surface of the dielectric layer 160) in the vicinity of the activated EW electrode 232. The increased Coulombic force can be sufficient to overcome the cohesive forces between molecules of a nearby droplet, effectively reducing the hydrophobicity of the EW surface 184 in the vicinity of the activated EW electrode 232. The foregoing can move the droplet on the EW surface 184.

Many EW electrodes (like 232) can be selectively activated and deactivated over some, most, or the entirety of the electrowetting surface 184. By selectively activating and deactivating such EW electrodes (like 232), droplets of liquid medium 214 or another liquid (not shown) in the second liquid medium 214 can be moved along the electrowetting surface 184. Equipment 132 (see Figure 1A) can control activation and deactivation of such EW electrodes (e.g., 232). As will be seen, such EW electrodes (like 232) can be fixed in a particular location, in the manner of conventional electrodes (e.g., metal electrodes), phototransistors, or photo-actuated electrodes. Alternatively, EW electrodes (like 232) can be virtual electrodes that are located at positions where electromagnetic radiation is incident on a photoconductive material, as occurs when light of an appropriate frequency is incident on a layer of amorphous silicon that is connected to a biasing electrode (like 158).

Figures 3-7 illustrate examples of the DEP configuration 122 and the EW configuration 124 of the enclosure 102.

In the examples shown in Figure 3, the structure 104 of the enclosure 102 can comprise a layer 352 of dielectric material, an electrode activation substrate 362, and a biasing electrode 372. The first surface 182 can be a surface of the electrode activation substrate 362, and the electrowetting surface 184 can be an outer surface of the dielectric layer 352, which can be hydrophobic. As also shown, the cover 110 can comprise a DEP biasing electrode 312 and an EW biasing electrode 314. The cover 110 can also include a layer 322 of electrically insulating material, which can extend across the DEP section 122 and the EW section 124 as illustrated. Alternatively, layer 322 can be disposed in the EW section 124 without extending into the DEP section 122, and of course, the layer 322 need not be present in some embodiments. The hydrophobic surface 165 can be an outer surface of the layer 322, which can be hydrophobic. The DEP biasing device 202 can be connected to the DEP biasing electrode 312 and the biasing electrode 372, and the EW biasing device 204 can be connected to the EW biasing electrode 314 and the biasing electrode 372.

Generally as shown in Figure 3, each of the dielectric layer 352, the electrode activation substrate 362, and the biasing electrode 372 can be a continuous layer or substrate that extends across both the DEP section 172 and the EW section 174 of the chamber 112. For example, each of the dielectric layer 352, the electrode activation substrate 362, and the biasing electrode 372 can be a continuous layer or substrate that extends across substantially the entirety of the structure 104. As also shown, the electrically insulating layer 322 of the cover 110 can also be a continuous layer that extends through both the DEP section 172 and the EW section 174 of the chamber 112. Figure 3 depicts the DEP biasing electrode 312 and the EW biasing electrode 314 of the cover 110 as two different unconnected electrodes each corresponding to one but not the other of the DEP section 172 or the EW section 174. The DEP biasing electrode 312 and the EW biasing electrode 314 can alternatively be a continuous biasing electrode like the biasing electrode 372. Similarly, any of the insulating layer 322, the dielectric layer 352, the electrode activation substrate 362, and/or the biasing electrode 372 can be two distinct structures each corresponding to one but not the other of the DEP section 172 or the EW section 174, as the DEP biasing electrode 312 and EW biasing electrode 314 are depicted in Figure 3. For example, the insulating layer 322 can be disposed only on the biasing electrode 314 in the EW section 124 but not on the biasing electrode 312 in the DEP section 122. The insulating layer 322 can comprise a hydrophobic material, or alternatively, a hydrophilic material examples of which can be as discussed above. Examples of the dielectric material 352 can also be as discussed above.

In the example shown in Figure 3, the DEP biasing electrode 312 is an example of the biasing electrode 166 in Figure 2. Similarly, the portion of the biasing electrode 372 to the left of the boundary 126 in Figure 3 is an example of the biasing electrode 156 in Figure 2, and the portion of the electrode activation substrate 362 to the left of the boundary 126 is an example of the DEP section 152 in Figure 2. Likewise, the EW biasing electrode 314 in Figure 3 is an example of the biasing electrode 168 in Figure 2; the portion of the electrode activation substrate 362 to the right of the boundary 126 in Figure 3 is an example of the EW section 154 in Figure 2; the portion of the dielectric layer 352 in Figure 3 to the right of the boundary 126 is an example of dielectric layer 160 in Figure 2; and the portion of the insulating layer 322 in Figure 3 to the right of the boundary 126 is an example of the layer 164 in Figure 2.

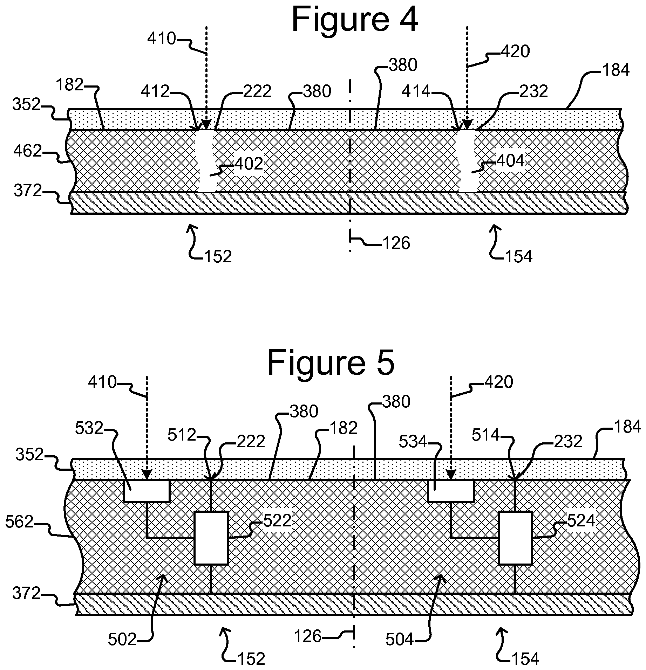

In the example shown in Figure 2, the EW section 154 but not the DEP section 152 of the structure 104 is illustrated as comprising a dielectric layer 160, yet the example shown in Figure 3 shows the dielectric layer 352 extending across both the DEP configuration 122 and the EW configuration 124 of the enclosure 102. In some embodiments, the thickness *t* of the dielectric layer 352 can be sufficiently thin that a DEP electrode like 222 (see Figure 2) activated at an outer surface 380 of the electrode activation substrate 362 (e.g., at the region 412 in Figure 4 or the region 512 in Figure 5) can effectively form an electrical connection through the dielectric layer 352 with the first medium 212 in the first chamber section 172 of the enclosure 104. Alternatively, or in addition, the DEP biasing device 202 can be operated such that the capacitive effect of the portion of the dielectric layer 352 to the left of the boundary 126 in Figure 3 is effectively shorted, and the EW biasing device 204 can be operated such that the capacitive effect of the portion of the dielectric layer 352 to the right of the boundary 126 is not shorted.

For example, the portion of the dielectric layer 352 to the left of the boundary 126 in Figure 3 can form a first effective capacitor (not shown) between the liquid medium 212 in the first chamber section 172 and any relatively high electrical conductivity region (e.g., like a DEP electrode 222 in Figure 2) formed at the outer surface 380 of the electrode activation substrate 362. Similarly, the portion of the dielectric layer 352 to the right of the boundary 126 in Figure 3 can form a second effective capacitor (not shown) between the liquid medium 214 in the second chamber section 174 and any relatively high electrical conductivity region (e.g., like an EW electrode 232) formed at the outer surface 380 of the electrode activation substrate 362. The DEP biasing device 202 can be operated at a frequency *f*₁ that is sufficiently high to effectively short the first effective capacitor (not shown) and thus effectively eliminate the capacitive effect of the portion of the dielectric layer 352 to the left of the boundary 126 in Figure 3. The EW biasing device 204, however, can be operated at a lower frequency *f*₂, which can be a frequency at which the capacitive effect of the second effective capacitor (not shown) is significant.

The apparatus 100 can be operated in a DEP mode in which, for example, the switch 206 is closed, thereby connecting the DEP biasing device 202 to the biasing electrodes 312, 372, but the switch 208 is open, thereby disconnecting the EW biasing device 204 from the biasing electrodes 314, 372. The apparatus 100 can similarly be operated in an EW mode in which the switch 206 is open but the switch 208 is closed. The equipment 132 (see Figure 1A) can control the switches 206, 208.

The electrode activation substrate 362 can be configured such that the DEP electrodes (like 222) and the EW electrodes (like 232) (see Figure 2) are virtual electrodes and/or fixed electrodes. Figure 4 illustrates an example in which the electrode activation substrate 362 comprises photoconductive material 462, and the DEP electrode 222 and the EW electrode 232 are virtual electrodes. Figure 5 shows an example in which the electrode activation substrate 362 comprises a circuit substrate 562, and the DEP electrode 222 and the EW electrode 232 are fixed.

As noted, in the example shown in Figure 4, the electrode activation substrate 362 can comprise photoconductive material 462, which can be a material that has a relatively high electrical impedance except when exposed directly to light. Examples of photoconductive material include semiconductor materials such as amorphous silicon. As shown, when light 410 is directed onto a relatively small region 412 of the photoconductive material 462 of the DEP section 152 of the structure 104, a relatively high electrically conductive path 402 is formed at the region 412 through the photoconductive material 462 to the biasing electrode 372. The conductive path 402 corresponds to the path 252 in Figure 2, and the light 410 thus activates a virtual DEP electrode 222 at the region 412.

As also shown in Figure 4, light 420 directed onto a relatively small region 414 of the EW section 154 of the structure 104 can similarly create a relatively high electrically conductive path 404 at the region 414 through the photoconductive material 462 to the biasing electrode 372. The conductive path 404 corresponds to the path 254 in Figure 2, and the light 420 thus activates a virtual EW electrode 232 at the region 412.

In the embodiment shown in Figure 4, DEP electrodes (like 222) can be activated in any desired pattern anywhere on the photoconductive material 462 by directing light 410 in the desired pattern onto the photoconductive material 462. Such DEP electrodes 222 can be deactivated by removing the light 410. EW electrodes (like 232) can similarly be activated and deactivated in any desired pattern anywhere on the photoconductive material 462 in accordance with a pattern of the light 414. The DEP electrodes (like 222) and the EW electrodes (like 232) are thus virtual electrodes. The DEP module 142 of Figure 1A can comprise a light source (not shown), and the DEP module 142 and/or the master controller 134 can control the light source to direct changing patterns of light into the apparatus 100 to selectively activate and deactivate such DEP electrodes (like 222) and EW electrodes (like 232) anywhere on the photoconductive material 462.

In the example shown in Figure 5, the electrode activation substrate 362 can comprise a circuit substrate 562, which can comprise a base material that has a relatively high electrical impedance but includes circuits for making relatively high electrical conductivity connections through the substrate. For example, a DEP electrode circuit 502 in the DEP section 152 of the structure 104 can comprise a switch 522 that provides a relatively high electrical conductivity connection (corresponding to the path 252 in Figure 2) from a relatively small fixed region 512 through the substrate 562 to the biasing electrode 372. The switch 522 can be selectively opened and closed to thereby selectively create a relatively high electrical impedance path from the region 512 to the biasing electrode 372 or a relatively high electrical conductivity path. In the example shown in Figure 5, the switch 522 is controlled by a photo element 532, which can open and close the switch 522 in response to a directed light beam 410. Alternatively, the switch 522 can be controlled by an external control module (e.g., the DEP module 142 of Figure 1A) via a control input (not shown). DEP electrode circuits like circuit 502 can be provided throughout the DEP section 152 of the structure 104, and a pattern of fixed DEP electrodes (like 222) can thus be provided through the DEP section 152. Such fixed DEP electrodes 222 can be activated and deactivated with light 410 or through external (e.g., electrical) control.

The DEP module 142 of Figure 1A can comprise a light source (not shown), and the DEP module 142 and/or the master controller 134 can control the light source to direct changing patterns of light 410 into the apparatus 100 to selectively activate and deactivate photo-actuated DEP electrodes (like 222 in Figs. 4 and 5). Alternatively, if some or all of the DEP electrodes are hardwired, the DEP module 142 and/or the master controller 134 can selectively control activation and deactivation of such DEP electrodes (like 222) in changing patterns.

The EW section 154 of the structure 104 can include similar EW electrode circuits 504. For example, an EW electrode circuit 504 in the EW section 154 of the structure 104 can comprise a switch 524 that provides a high conductivity electrical connection (corresponding to the path 254 in Figure 2) from a relatively small fixed region 514 through the substrate 562 to the biasing electrode 372. The switch 524 can be selectively opened and closed to thereby selectively create a relatively high electrical impedance path from the region 514 to the biasing electrode 372 or a relatively high electrical conductivity path. In the example shown in Figure 5, the switch 524 is controlled by a photo element 524, which can open and close the switch 524 in response to a directed light beam 420. Alternatively, the switch 524 can be controlled by an external control module (e.g., the EW module 144 of Figure 1A) by an electrical control input (not shown). EW electrode circuits like circuit 504 can be provided throughout the EW section 154 of the structure 104, and a pattern of fixed EW electrodes (like 232) can thus be provided throughout the EW section 154. Such EW electrodes can be activated and deactivated with light 412 or through external control.

The EW module 144 of Figure 1A can comprise a light source (not shown), and the EW module 144 and/or the master controller 134 can control the light source to direct changing patterns of light 420 into the apparatus 100 to selectively activate and deactivate photo-actuated EW electrodes (like 232 in Figs. 4 and 5). Alternatively, if some or all of the DEP electrodes are hardwired, the EW module 144 and/or the master controller 134 can selectively control activation and deactivation of such EW electrodes (like 232) in changing patterns.

In some embodiments, switch 522 and/or switch 524 in Figure 5 can comprise a transistor. For example, switch 522 and/or switch 524 can comprise a transistor that can be activated and deactivated by photo element 532 and/or 534. Alternatively, switch 522 and/or 534 configured as a transistor can be activated and deactivated by a hardwired control connection (not shown). As yet another example, switch 522 and/or switch 524 can comprise a photo transistor activated by directing light 410 or 420 onto the photo transistor itself and deactivated by removing the light 410 or 420 from the phototransistor. If the switch 522 and/or 524 is configured as a hardwired transistor or a photo transistor, there may be no need for photo element 532 or 534. In some embodiments, the DEP electrode 222 in Figure 5 can comprise a fixed physical electrode at region 512 to which the switch 522 is electrically connected. The EW electrode 232 can similarly comprise a fixed physical electrode at region 514 to which the switch 524 is electrically connected.

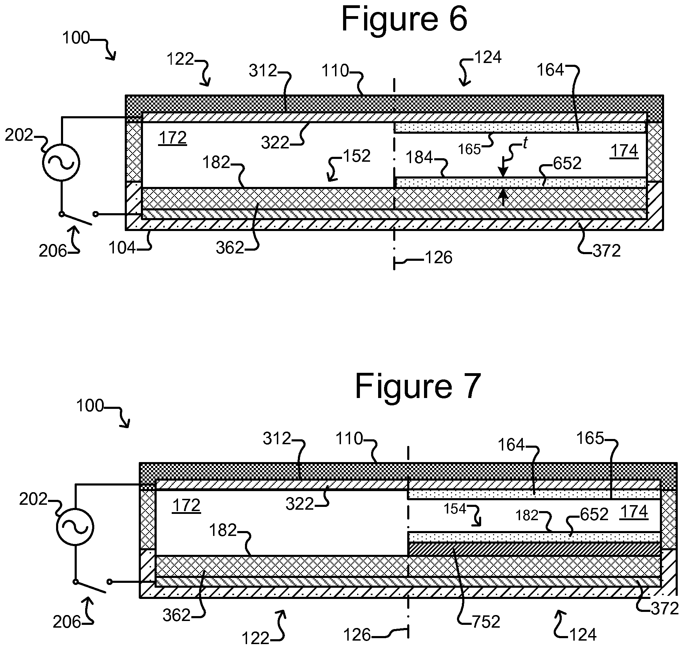

As noted, Figures 6 and 7, like Figure 3, illustrate example configurations of the DEP configuration 122 and EW configuration 124 of the enclosure 102.

The configuration illustrated in Figure 6 is similar to Figure 3 except that a dielectric layer 652 replaces the dielectric layer 352. The dielectric layer 652 forms the electrowetting surface 184 of the second chamber section 174 but not the first surface 182 of the first chamber section 172. Thus, the dielectric layer 652 is part of the EW configuration 124 of the enclosure 104 but not the DEP configuration 122. Because the dielectric layer 652 does not extend across the first surface 182 of the DEP configuration 122, the thickness *t* of the dielectric layer 652 can be greater than the thickness *t* of the dielectric layer 352 in Figure 3. Otherwise, the dielectric layer 652 can be like and can comprise the same materials as the dielectric layer 352.

The configuration of Figure 7 is similar to Figure 6 except the configuration of Figure 7 includes an additional dielectric layer 752 between the dielectric layer 652 and the electrode activation substrate 362. The dielectric layer 652 and the dielectric layer 752 can be part of the EW configuration 124 of the enclosure 104, but those layers are not part of the DEP configuration 122. The dielectric layer 752 can be like and can comprise the same materials as any dielectric layer (e.g., 352) mentioned herein.

Although not shown in Figure 7, a biasing electrode can be located in the EW section 124 between the additional dielectric layer 752 and the portion of the electrode activation substrate 362 that is in the EW section 124. The biasing device 204 (see Figure 2) can be connected to the portion of the biasing electrode 312 (which can be bifurcated and thus comprise a portion in the DEP section 122 and a separate electrically isolated portion in the EW section 124) that is to the right of the boundary 126 in Figure 7 and the biasing electrode (not shown) between the additional dielectric layer 752 and the portion of the electrode activation substrate 362 in the EW section 124 rather than to the biasing electrode 372.

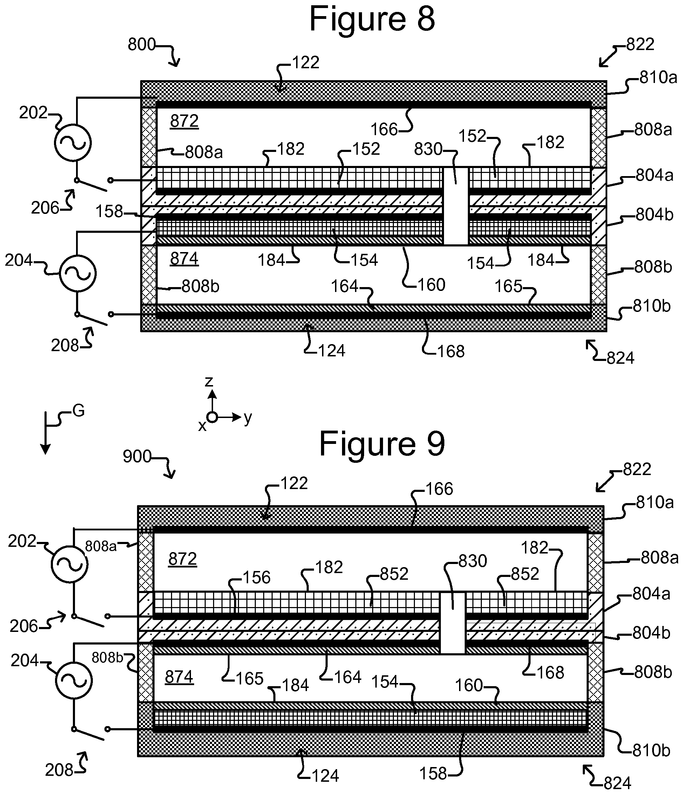

Figures 1A-1C show the first chamber section 172 and the second section 174 of the enclosure 104 side-by-side (e.g., substantially in a same plane). The foregoing, however, is merely an example, and other configurations are possible. Figure 8 illustrates an example in which such sections are stacked.

Figure 8 illustrates a microfluidic apparatus 800 that can comprise a first sub-enclosure 822 stacked on a second sub-enclosure 824. For example, each sub-enclosure 822, 824 can comprise a structure 804, a fluidic circuit frame 808, and a cover 810 each of which can be the same as or similar to the structure 104, fluidic circuit frame 108, and cover 110 of Figures 1A-1C. Although two stacked sub-enclosures 822, 824 are shown in Figure 8, there can be more such stacked sub-enclosures.

Either or all of the sub-enclosures 822, 824 can be configured as a DEP configured device and/or an EW configured device. That is, although the first sub-enclosure 822 is illustrated as comprising a DEP configuration 122 and the second sub-enclosure 824 is shown as comprising an EW configuration 124, both sub-enclosures 822, 824 can comprise a DEP configuration (e.g., like 122) or an EW configuration (e.g., like 124). As yet another alternative, one or both of the sub-enclosures 822, 824 can be configured in part as a DEP configuration and in part as an EW configuration (e.g., one or both of the sub-enclosures 822, 824 can be configured like the apparatus 100 shown in Figures 1A-2).

As illustrated in Figure 8, the first enclosure 822 can comprise a DEP configuration 122, and the second enclosure 824 can comprise an EW configuration 124 as discussed above. For example, the structure 804a of the first enclosure 822 can comprise the DEP section 152, including a first surface 182, and the cover 810a can comprise the biasing electrode 166, as discussed above. Similarly, the structure 804b of the second enclosure 822 can comprise the EW section 154, the dielectric layer 160, and the electrowetting surface 184, and the cover 810b can comprise the hydrophobic surface 165, the layer 164, and the biasing electrode 168, as discussed above.

The first sub-enclosure 822 can define a first section 872 for holding a liquid medium (e.g., the first liquid medium 212 shown in Figure 2), and the DEP configuration 122 can select and manipulate micro-objects (e.g., like 228 in Figure 2) in such a liquid medium in the first section 872. The second sub-enclosure 824 can similarly define a second section 874 for holding a liquid medium (e.g., the second liquid medium 214 shown in Figure 2), and the EW configuration 124 can manipulate a liquid medium on the electrowetting surface 184, as discussed above, in the second section 874. As also shown, there can be one or more passages 830 (one is shown but there can be more) from the first section 872 to the second section 874. The sidewalls of such a passage 830 can be hydrophilic in which case an aqueous medium in the first section 872 can naturally enter and fill the passage 830. Alternatively, the sidewalls of the passage 830 can be hydrophobic.

Figure 9 illustrates another example of a microfluidic apparatus 900 that can be generally similar to the device 800 except that the positions of the biasing electrode 168, layer 164, and hydrophobic surface 165, on one hand, and the electrowetting surface 184, dielectric layer 160, EW section 154, and biasing electrode 158 are different (e.g., opposite) than the positions shown in Figure 8.

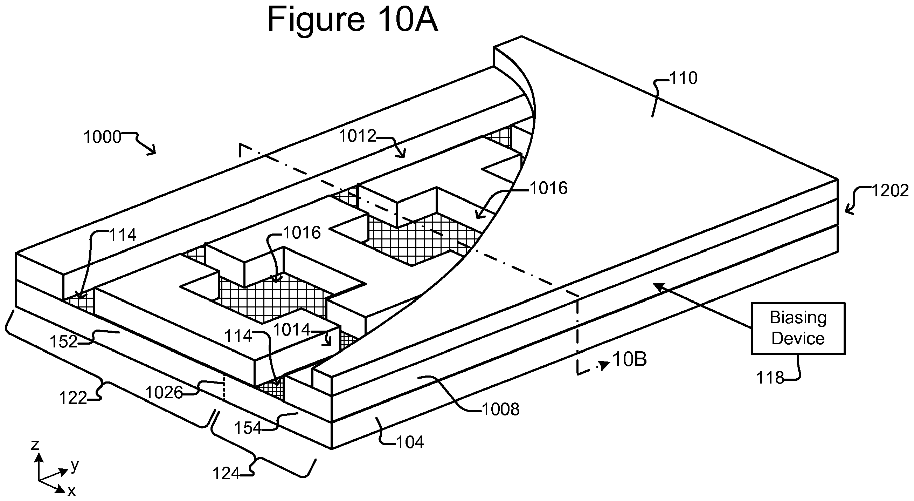

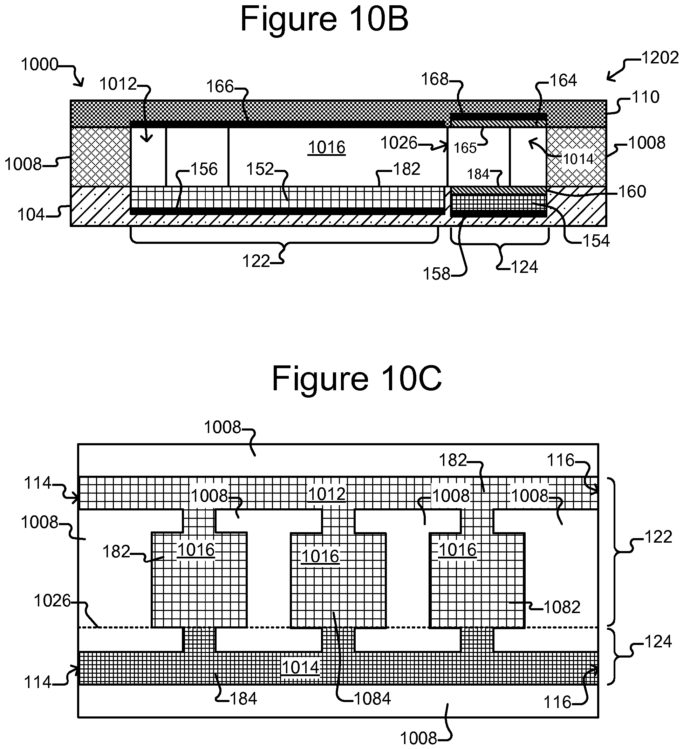

As mentioned, the configuration of the apparatus 100 shown in Figures 1A-1C as comprising a chamber 112 divided into a first chamber section 172 and a second chamber section 174 is an example, and many other configurations are possible. Figures 10A-10C illustrate an example of a microfluidic apparatus 1000 comprising multiple fluidic channels 1012, 1014 (two are shown but there can be more) and multiple holding pens 1016 (three are shown but there can be fewer or more) each of which can be connected to one or more of the channels 1012, 1014.

The apparatus 1000 can be generally similar to the apparatus 100, and like numbered elements in Figures 10A-10C can be the same as in Figures 1A-1C. The fluidic circuit frame 1008 of the apparatus 1000, however, can define, with the structure 104 and the cover 110, a first channel 1012, a second channel 1014, and holding pens 1016, which as shown, can be connected to the channels 1012, 1014. Otherwise, the fluidic circuit frame 1008 can be the same as or similar to the fluidic circuit frame 108.

In the example shown in Figures 10A-10C, the first channel 1012 and the pens 1016 can be configured to hold a first liquid medium (not shown but can be the first liquid medium 212 of Figure 2), and the structure 104 and cover 110 can include the DEP configuration 122 for selecting and manipulating micro-objects in the first liquid medium. For example, the structure 104 can comprise the biasing electrode 156, DEP section 152, and first surface 182, and the cover 110 can comprise the biasing electrode 166, all of which can be as discussed above. Similarly, the structure 104 can also comprise the biasing electrode 158, EW section 154, dielectric layer 160, and electrowetting surface 184, and the cover 110 can also comprise the hydrophobic surface 165, layer 164, and biasing electrode 168, all of which can be as discussed above. As discussed above, the DEP configuration 122 can be for selecting and manipulating micro-objects (e.g., 228) in a first liquid medium (e.g., 212) on the first surface 182 in the first channel 1012 and pens 1016, and the EW configuration 124 can be for manipulating a liquid medium (not shown) on the electrowetting surface 184 in the second channel 1014.

In Figures 10A-10C, the boundary 1026 can be the same as the boundary 126 in Figures 1A-1C: the boundary 1026 is the boundary between the first surface 182 and the electrowetting surface 184, which can be the boundary between a first section (comparable to the first chamber section 172 of Figures 1A-1C) comprising the first channel 1012 and the pens 1016 and a second section (comparable to the second chamber section 174 of Figures 1A-1C) comprising the second channel 1014.

Although not shown in Figures 10A-10C or in Figures 8 and 9, the equipment 132 and biasing device 118 (e.g., comprising the biasing devices 202, 204 and switches 206, 208 of Figure 2) of Figures 1A-1C can bias, control, and provide miscellaneous functions to the devices 800, 900, and 1000 of Figures 8-10C.

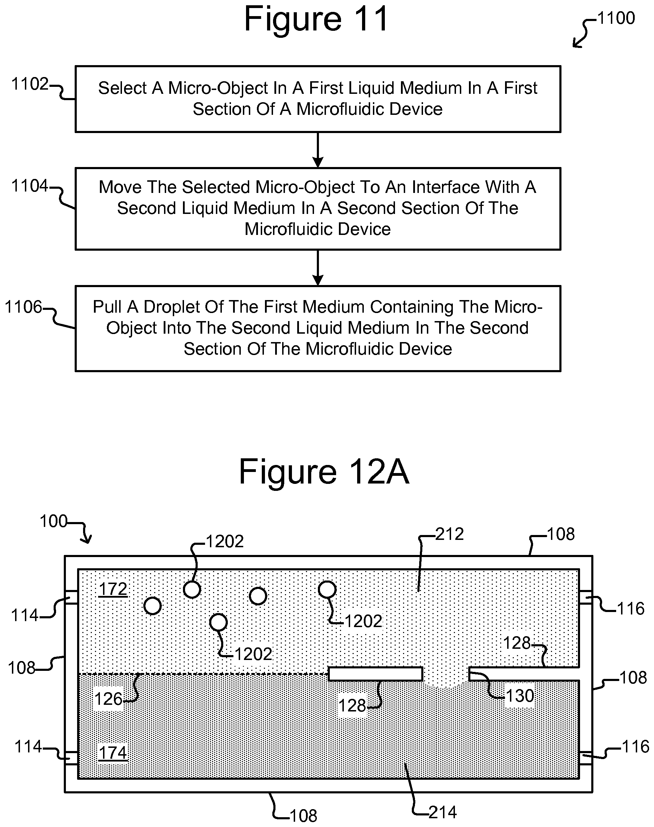

Figure 11 is an example of a process 1100 for moving a micro-object from a first liquid medium in a microfluidic apparatus to a second liquid medium. For ease of illustration and discussion, the process 1100 is discussed below with respect to the apparatus 100 of Figures 1A-1C and the apparatus 800 of Figure 8. The process 1100 is not so limited, however, but can be performed on other microfluidic apparatuses such as the apparatus 900 of Figure 9, the apparatus 1000 of Figures 10A-10C, or other such devices.

As shown, at step 1102 of process 1100, a micro-object in a DEP configured portion of a microfluidic apparatus can be selected. Figures 12A-15 illustrates examples.

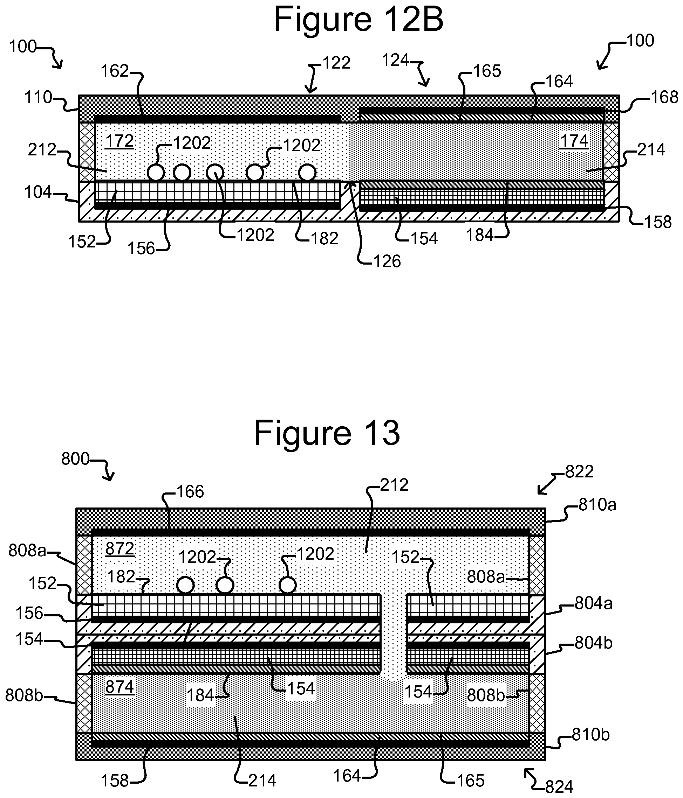

Figure 12A shows a top view of the apparatus 100, with the cover 110 removed; and Figure 12B is a across-sectional side view of the apparatus 100, corresponding to Figures 1C and 1B but with the first liquid medium 212 in the first chamber section 172 of the enclosure 102 and the second liquid medium 214 in the second chamber section 174 of the enclosure 102 (as illustrated in Figure 2). In addition, micro-objects 1202 (which can be like the micro-object 218 of Figure 2) can be suspended in the first liquid medium 212 in the first chamber section 172. Figure 13 shows the device 800 of Figure 8 with the first liquid medium 212 in the first section 872 of the first sub-enclosure 822 and the second liquid medium 214 in the second section 874 of the second sub-enclosure 824. Micro-objects 1202 are also shown in the first medium 212 in the first section 872.

Although not shown in Figures 12A-21, the equipment 132 and biasing device 118 (e.g., comprising the biasing devices 202, 204 and switches 206, 208 of Figure 2) of Figures 1A-1C can bias, control, and provide miscellaneous functions to the devices 100 and 800 illustrated in Figures 12A-21. Indeed, the master controller 134 can be configured to perform one, some, or all of the steps of the process 1100.

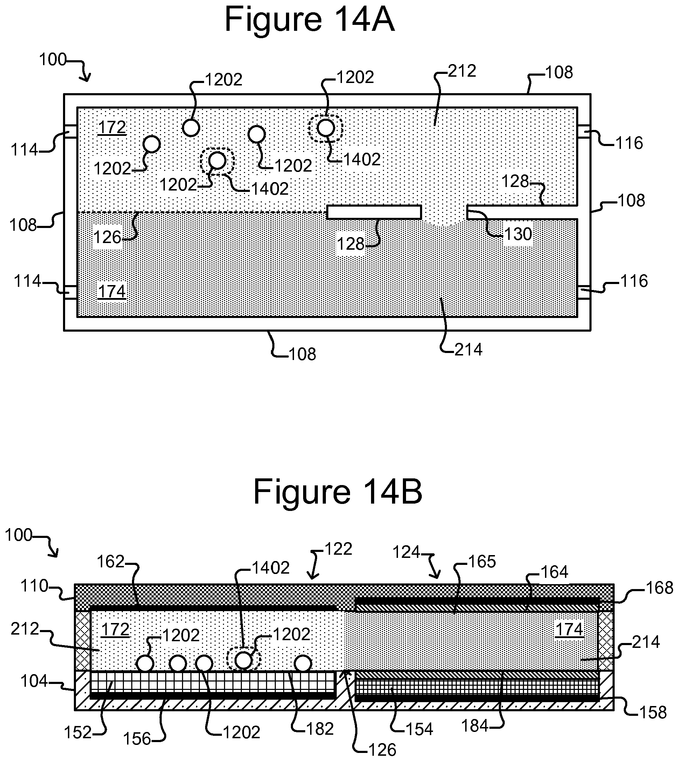

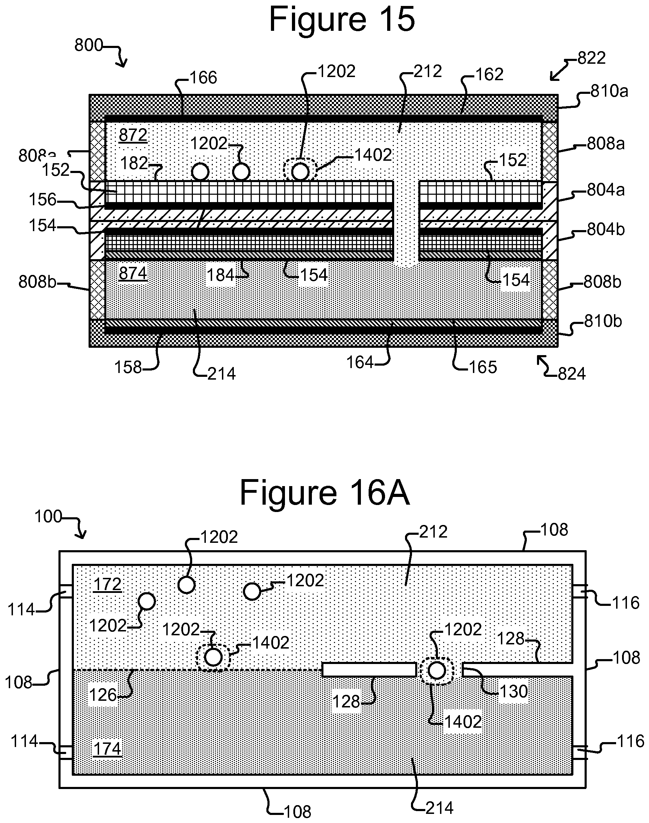

As shown in Figures 14A and 14B, one or more of the micro-objects 1202 in the first liquid medium 212 can be selected and captured with a DEP trap 1402. The DEP traps 1402 can be created by activating one or more DEP electrodes 222 (not shown in Figures 14A and 14B) at the first surface 182 of the DEP section 152 (as discussed above with respect to Figure 2) in a pattern that surrounds the selected micro-object 1202, thereby capturing the micro-object 1202. A specific one or more of the micro-objects 1202 can be identified and selected from a group of micro-objects 1202 in the first chamber section 172 based on any of a number of characteristics (e.g., cell size and/or morphology, nuclear size and/or morphology, cell surface markers, cell secretions, and the like). Similarly, as shown in Figure 15, one or more specific micro-objects 1202 can be identified and selected with a DEP trap 1402 in the first section 872 of the device 800.

Returning again to Figure 11, at step 1104 of process 1100, one or more micro-objects selected at step 1102 can be moved to an interface with the second liquid medium in the device. Figures 16A-17 illustrate examples.

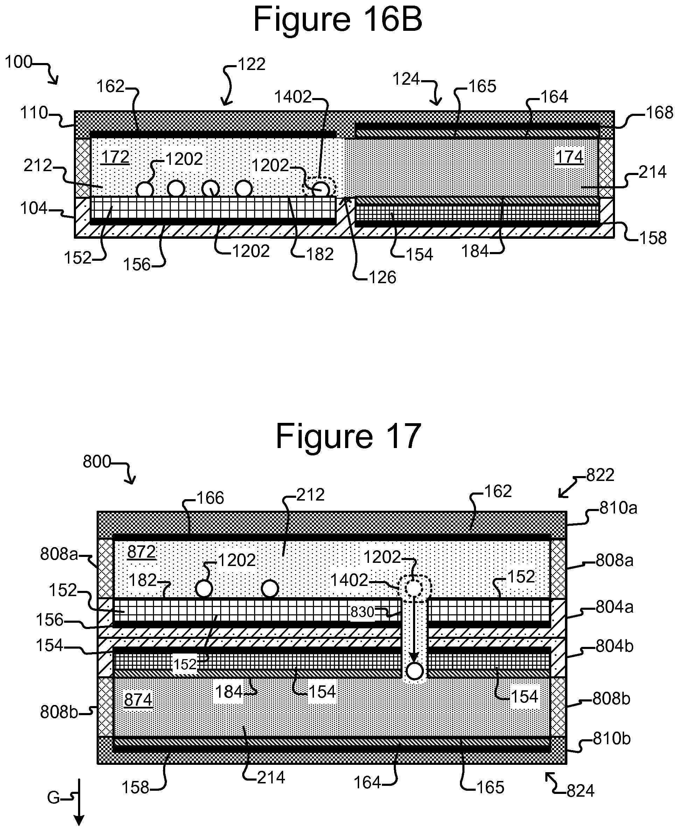

As shown in Figure 16A, a selected micro-object 1202 can be moved in the apparatus 100 to the passage 130 through the physical barrier 128. Alternatively, a selected micro-object 1202 can be moved to a portion of the boundary 126 that does not have a physical barrier. The selected micro-objects 1202 can be moved in the first liquid medium 212 in the first chamber section 172 in the apparatus 100 by moving the traps 1402, which can be accomplished by activating and deactivating DEP electrodes 222 (not shown in Figures 16A and 16B) on the first surface 182 of the DEP section 152 as discussed above. The movement of the selected micro-objects 1202 can involve tilting the apparatus 100 such that the force of gravity (G) pulls the micro-objects 1202 towards the boundary 126 or passage 130. In certain embodiments, the micro-objects 1202 can be moved towards the boundary 126 or passage 130 (e.g., by tilting the apparatus and allowing gravitational force to act upon the micro-objects 1202) prior to the micro-objects 1202 being selected.

As still another example illustrated in Figure 17, a selected micro-object 1202 in the first section 872 of the device 800 can be moved to the passage 830, where the selected micro-object 1202 can be released into the passage 830. The selected micro-objects 1202 can be moved to the passage 830 by moving the trap 1402 to the passage, which can be accomplished by activating and deactivating DEP electrodes 222 (not shown in Figure 17) on the first surface 182 of the DEP section 152, as discussed above with respect to Figure 2. The selected micro-object 1202 can be released by deactivating DEP electrodes 222 of the trap 1402. Again, the movement of the selected micro-objects 1202 can involve tilting the apparatus 800 such that the force of gravity (G) pulls the micro-objects 1202 towards the passage 830, as discussed above.

The force of gravity (G) can move the released micro-object 1202 to the bottom of the passage 830, which is located at the interface with the second liquid medium 214 in the second section 874. Alternatively, the released micro-object 1202 can be moved down the passage 830 by forces other than gravity. For example, a flow of the first liquid medium 212 in the passage 830 can move the released micro-object 1202 down the passage 830. As another example, the micro-object 1202 can be moved down the passage 830 by the DEP trap 1402.

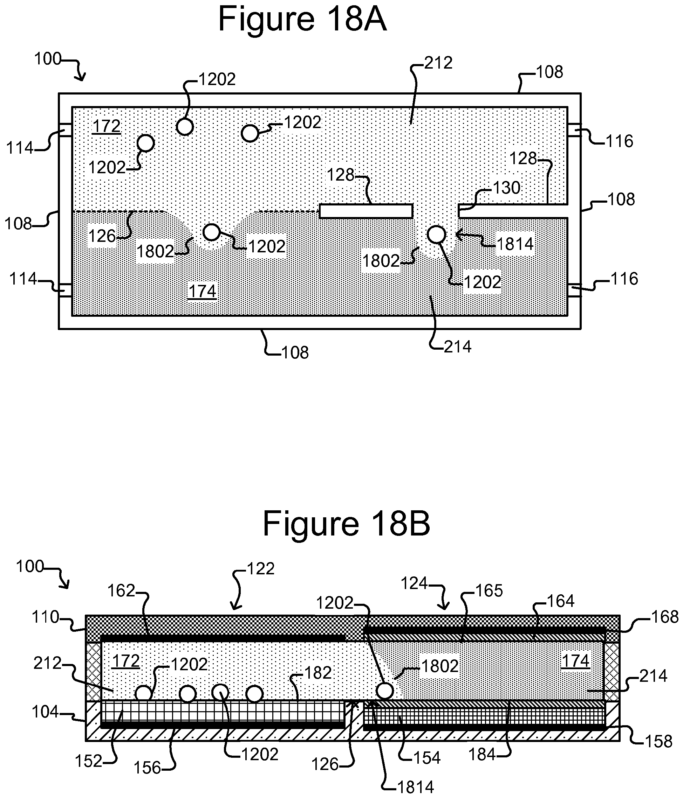

Referring again to Figure 11, at step 1106 of process 1100, a droplet of the first liquid medium containing the micro-object from the first liquid medium 212 can be pulled into the second medium. Figures 18A-19 illustrate examples.

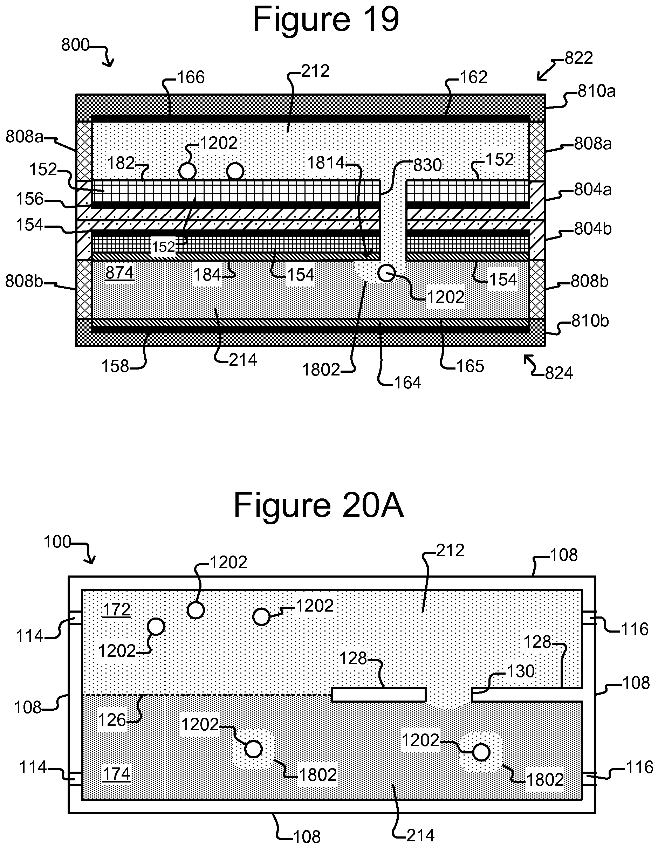

As shown in Figure 18A, a droplet 1802 of the first liquid medium 212 with a micro-object 1202 can be pulled from the first chamber section 172, through the passage 130 in the physical barrier 128 of the apparatus 100, and into the second liquid medium 214 in the second chamber section 174 of the apparatus 100. As another example illustrated in Figures 18A and 18B, a droplet 1802 can be pulled into the second medium 214 from the first medium 212 across a portion of the boundary 126 where there is no physical barrier 128. Regardless, a droplet 1802 of the first liquid medium 212 can be pulled from the first chamber section 172 into the second liquid medium 214 in the second chamber section 174 by activating EW electrodes 232 (not shown in Figures 18A and 18B) on the electrowetting surface 184 in a region 814 adjacent the boundary 126 between the first and second liquid media 212, 214, generally as discussed above with respect to Figure 2. As noted in the discussion of Figure 2 above, active EW electrodes 232 on the electrowetting surface 184 can attract the first liquid medium 212 and thereby move a droplet of the first liquid medium 212 along the electrowetting surface 184. Another example is shown in Figure 19, which shows an example of drawing a droplet 1802 of the first medium 212 from the passage 830 into the second medium 214 in the second section 874.

Additional actions can be taken to aid in pulling a droplet 1802 from the first chamber section 172 into the second chamber section 174. For example, a pressure differential can be created that tends to draw a droplet 1802 from the first chamber section 172 into the second chamber section 174. Such a pressure differential can aid in pulling the droplet 1802 into the second chamber section 874 and can thus be utilized in conjunction with activating EW electrodes 232 as discussed above. Such a pressure differential can be induced hydrodynamically, by a piezo device, utilizing air pressure, utilizing liquid pressure, or the like. Rather than aiding in pulling a droplet 1802 into the second chamber section 174, inducing a pressure differential can be utilized to pull the droplet 1802 into the second chamber section 174 without activating EW electrodes 232. Pressure and/or other techniques can thus be utilized to aid in pulling a droplet 1802 into the second chamber section 174, or such techniques can be utilized by themselves to pull a droplet 1802 into the second chamber section 174 without activating EW electrodes 232.

Although not shown in Figures 18A and 18B, additional elements can be included. For example, a moveable cutting tool (e.g., comprising a knife blade) can be provided in the chamber 112 and configured to separate a droplet 1802 in the second chamber section 174 from the medium 212 in the first chamber section 172.

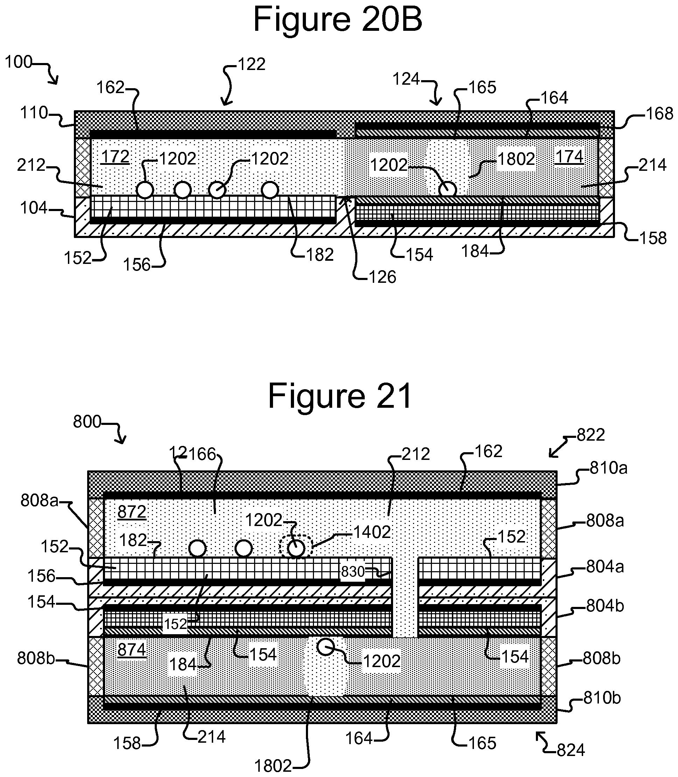

As shown in Figures 20A and 20B, the droplets 1802 of the first liquid medium 212 pulled into the second medium 214 can be moved about (along with the micro-objects 1202 in the droplets 1802) in the second chamber section 174, which can be done by selectively activating and deactivating EW electrodes 232 (not shown in Figures 20A and 20B) at a region of the electrowetting surface 184 that is immediately adjacent (e.g., in front of) the droplet 1802, generally as discussed above with respect to Figure 2. As shown in Figure 21, the droplets 1802 can similarly be moved about in the second liquid medium 214 in the second section 874 of apparatus 800. For example, the droplets 1802 can be moved to other locations in or exported from the microfluidic device.

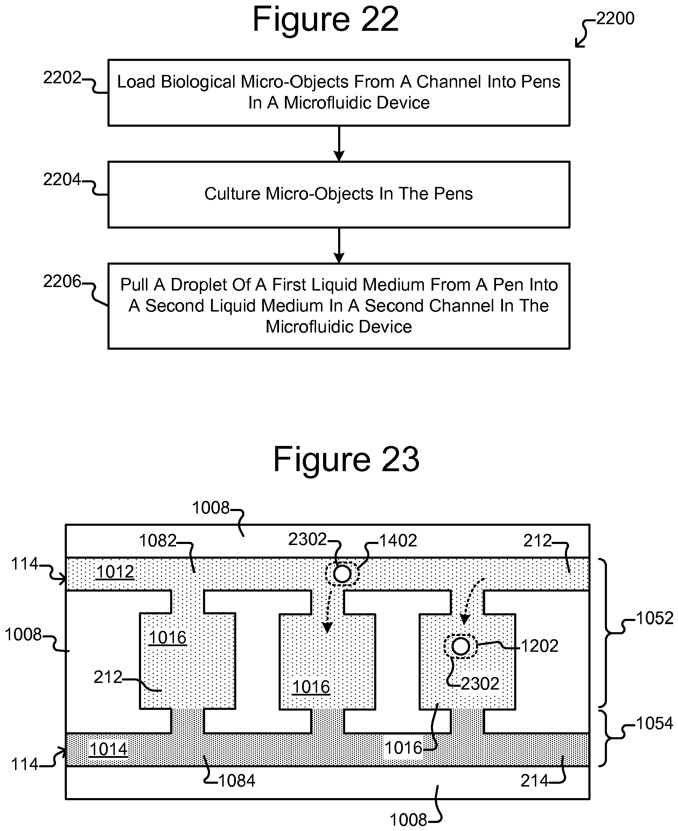

Figure 22 is an example of a process 2200 for culturing biological micro-objects in a microfluidic apparatus. For ease of illustration and discussion, the process 2200 is discussed below with respect to the apparatus 1000 of Figures 10A-10C. The process 2200 is not so limited, however, but can be performed with other microfluidic apparatuses.

Although not shown in Figures 23-25, the equipment 132 and biasing device 118 (e.g., comprising the biasing devices 202, 204 and switches 206, 208 of Figure 2) of Figures 1A-1C can bias, control, and provide miscellaneous functions to the apparatus 1000 illustrated in Figures 23-25. The master controller 134 can be configured to perform one, some, or all of the steps of the process 2200.

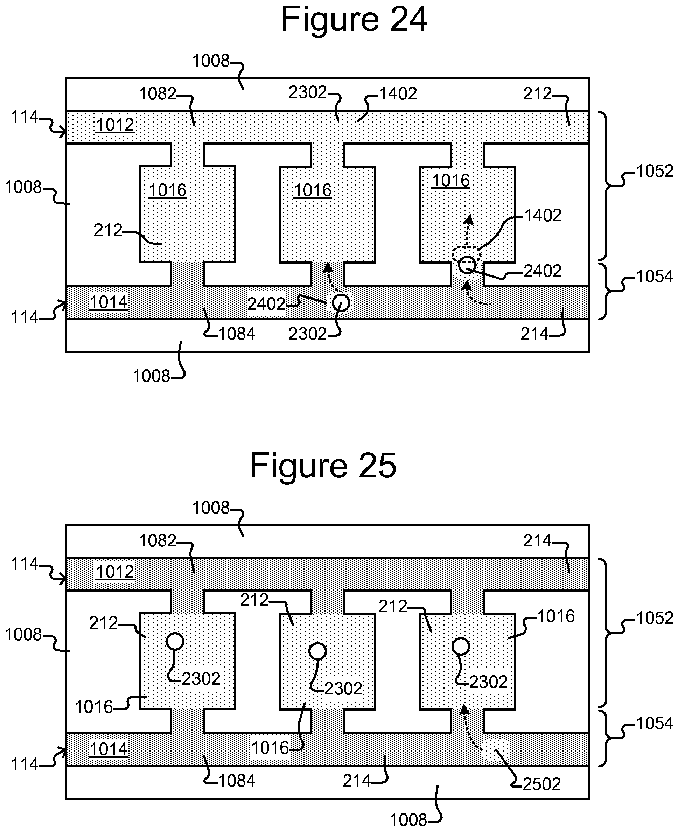

As shown, at step 2202 of process 2200, biological micro-objects can be loaded into holding pens in a micro-fluidic device. Examples are illustrated in Figures 23 and 24, which show top views of the apparatus 1000 of Figures 10A-10C, and in particular with the cover 110 removed as shown in Figure 10C. In Figures 23 and 24, the first channel 1012 and the pens 1016 contain the first liquid medium 212 and the second channel 1014 contains the second liquid medium 214.

As shown in Figure 23, biological micro-objects 2302 can be selected in the first channel 1012 and moved into the pens 1016. For example, a particular biological micro-object 2302 can be selected and moved by trapping the particular micro-object 2302 with a DEP trap 1402 and moving the DEP trap 1402 into a pen 1016, as discussed above with respect to Figure 11. The movement of the biological micro-objects 2302 can involve tilting the apparatus 1000 such that the force of gravity (G) pulls the biological micro-objects 2302 towards and/or into the pens 1016. In certain embodiments, the biological micro-objects 2302 can be moved towards and/or into the pens 1016 (e.g., by tilting the apparatus and allowing gravitational force to act upon the biological micro-objects 2302) prior to the biological micro-objects 2302 being selected.

In the example shown in Figure 24, biological micro-objects 2302 can be introduced (e.g., through an inlet 114) into the second channel 1014. As shown, one or more of the micro-objects 2302 can be inside droplets 2402 of a medium (e.g., the first medium 212) in the second channel 1014. Those droplets 2402 can be moved to openings of the pens 1016 generally as shown. The droplets 2402 can be moved in the second medium 214, generally as discussed above. Once a droplet 2402 is moved to an interface between the first medium 212 and the second medium 214 at an opening to a pen 1016, the one or more biological micro-objects 2302 can be moved from the droplet 2402 in the second medium 214 into the first medium 212 in the pen 1016. For example, the droplet 2402 at the interface between the first medium 212 and the second medium 214 can be merged with the interface by generating an electrowetting force at the boundary. Thereafter, DEP traps 1402 that attract a micro-object 2402 can optionally be generated in the DEP section 1052, which can thus attract a micro-object 2402 sufficiently to pull the micro-object 2402 away from the interface between the first medium 212 and the second medium 214.

Regardless of how the biological micro-objects 2302 are loaded into pens 1016 at step 2202, individual biological micro-objects 2302 can be placed into pens 1016 such that each of one or more of the pens 1016 contains a single cell. Of course, multiple biological micro-objects 2302 can be placed into one or more individual pens 1016.

As shown, at step 2204 of process 2200, the biological micro-objects 2302 in the pens 1016 can be cultured. For example, once one or more biological micro-objects 2302 are placed into each pen 1016, the micro-objects can be left for a time to grow, secrete biological material, divide, or the like. Nutrients can be provided to the biological micro-objects 2302 in the pens 1016 in a flow (not shown) of the first medium 212 in the first channel 1012. As another example, as shown in Figure 25, once biological micro-objects 2302 are in the pens 1016, the first liquid medium 212 can be replaced in the first channel 1012 with the second liquid medium 214. This can keep the micro-objects 2302 from escaping the pens 1016 into the first channel 1012. Nutrients can be provided to the micro-objects 2302 in the pens 1016 by moving droplets 2502 of the first liquid medium 212 through the second liquid medium 214 in the second channel 1014 into the pens 1016. Such droplets 2502 can contain nutrients for the micro-objects 2302 in the pens 1016. The droplets 2502 can be moved in the second channel 1014 in the same way that droplets 1802 are moved as discussed above with respect to Figures 18A-21.

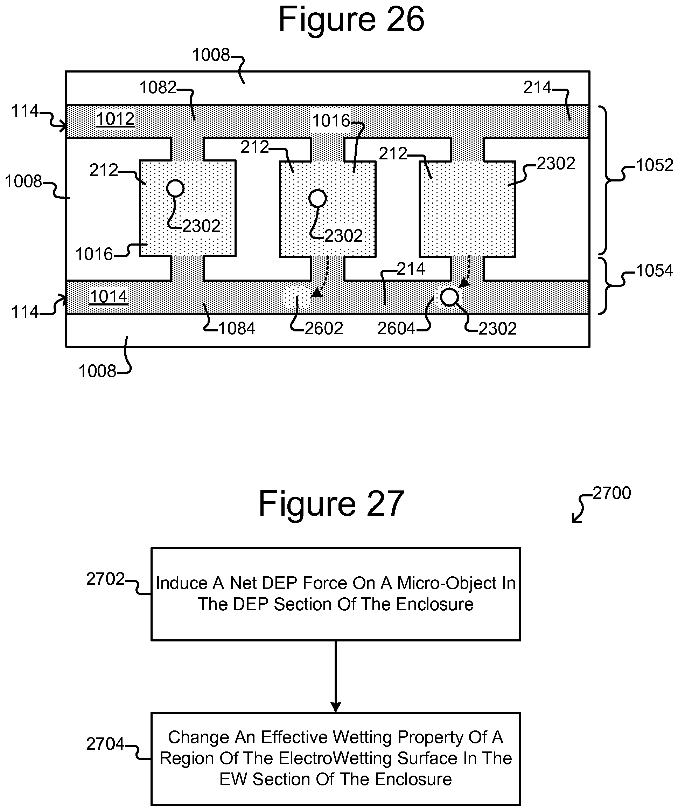

At step 2206 of process 2200, droplets of the first liquid medium can be pulled from the pens into the second channel. For example, as shown in Figure 26, an aliquot in the form of one or more droplets 2602 of the first liquid medium 212 can be pulled from a pen 1016 into the second liquid medium 214 in the second channel 1014. Such a droplet 2602 can then be moved in the second channel 1014 to a location where the droplet 2602 can be analyzed to determine the chemical or material content of the droplet 2602. The content of the first liquid medium 212 in any of the pens 1016 can thus be analyzed by removing one or more droplets 2602 form the pen 1016. The droplet 2602 can be pulled from a pen 1016 into the second channel 1014 and moved in the second liquid medium 214 in the second channel 1014 as discussed above with respect to 20A-21.

As another example, a droplet 2604 containing a biological micro-object 2302 can be pulled from a pen 1016 into the second channel 1014. This can be accomplished in accordance with the process 1100 performed in a pen 1016 and the second channel 1014.

Figure 27 illustrates an example of a process 2700 that can be performed on a microfluidic apparatus comprising at least one DEP section and at least one EW section. For example, the process 2700 can be performed on the microfluidic apparatus 100 of Figures 1A-1C or the apparatus 1000 of Figures 10A-10C.

As shown, at step 2702, a net DEP force can be induced on a micro-object in a DEP section of a microfluidic apparatus. For example, the net DEP force (F) can be induced on the micro-object 228 as illustrated in Figure 2 and discussed above. The net DEP force (F) can be sufficiently strong to move the micro-object 228 on the first surface 182. Generally as discussed above, the step 2702 can be repeated for different DEP electrodes 222 at the first surface 182 to move the micro-object 228 along any of a variety of possible paths across the surface 182.

At step 2704, an effective wetting property of a region of an electrowetting surface in an EW section of the microfluidic apparatus can be changed. For example, an effective wetting property of the electrowetting surface 184 at an EW electrode 232 can be changed as illustrated in Figure 2 and discussed above. The change can be sufficient to move liquid medium (e.g., a droplet of liquid medium) on the electrowetting surface 184. Generally as discussed above, the step 2704 can be repeated for different EW electrodes 232 at the electrowetting surface 184 to move the liquid medium (e.g., a droplet) along any of a variety of possible paths across the electrowetting surface 184.

The steps 2702 and 2704 can alternatively be performed in any manner discussed herein for inducing a net DEP force on a micro-object or changing an effective wetting property of an electrowetting surface. Moreover, the steps 2702 and 2704 can be performed simultaneously.

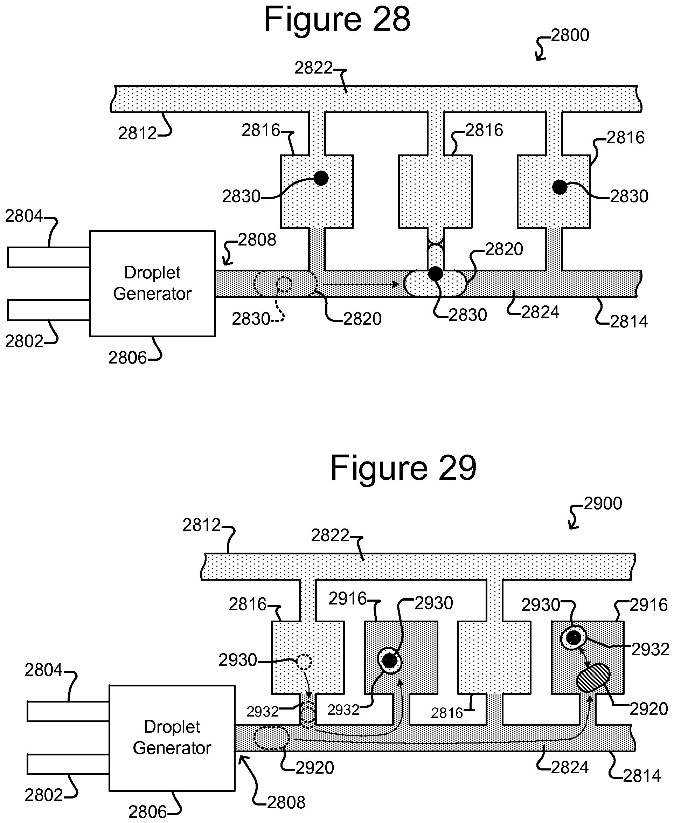

Figure 28 illustrates an example of a droplet generator 2806 for providing fluidic droplets to a microfluidic circuit 2800. In the example shown in Figure 28, the microfluidic circuit 2800 is illustrated as comprising a perfusion channel 2812, a sample channel 2814, and holding pens 2816, which can be fluidically connected to one or both of the channels 2812 and 2814. The perfusion channel 2812 and holding pens 2816 can comprise DEP configurations, and the sample channel 2814 can comprise an EW configuration. For example, the profusion channel 2812 and holding pens 2816 can be like the DEP channel 1012 and holding pens 1016 of Figures 10A-10C, and the sample channel 2814 can be like the EW channel 1014 of Figures 10A-10C. The microfluidic circuit 2800, however, is but an example, and the droplet generator 2806 can be utilized with other microfluidic circuits.

For example, the droplet generator 2806 can be utilized with microfluidic circuits that do not include DEP and/or EW configured sections. Regardless, the droplet generator 2806 and any microfluidic circuit to which it provides droplets can be part of a microfluidic device (either an integral part or connected thereto), which can be like any of the microfluidic devices illustrated in the drawings or described herein. Although one droplet generator 2806 is shown in Figure 28, more than one such droplet generator 2806 can provide droplets to the microfluidic circuit 2800.

The perfusion channel 2812 and the pens 2816 can be filled with a first fluidic medium 2822, and the sample channel 2814 can be filled with a second fluidic medium 2824. The first fluidic medium 2822 (hereinafter an "aqueous medium") can be an aqueous medium, such as a sample medium for maintaining, culturing, or the like biological micro-objects 2830. The second fluidic medium 2824 (hereinafter an "immiscible medium") can be a medium in which the aqueous medium 2822 is immiscible. Examples of the aqueous medium 2822 and the immiscible medium 2824 include any of the examples discussed above for various media.

As shown, the droplet generator 2806 can comprise one or more fluidic inputs 2802 and 2804 (two are shown but there can be fewer or more) and a fluidic output 2808, which can be connected to the sample channel 2814. Aqueous medium 2822, immiscible medium 2824, biological micro-objects 2830, reagents, and/or other biological media can be loaded through the inputs 2802 and 2804 into the droplet generator 2806. The droplet generator 2806 can generate and output into the channel 2814 droplets 2820 of the aqueous medium 2822 (which can, but need not, contain one or more biological micro-objects 2830), reagents, or other biological medium. If the channel 2814 is configured as an EW channel, the droplets 2820 can be moved in the channel 2814 utilizing electrowetting or optoelectrowetting as discussed above. Alternatively, the droplets 2820 can be moved in the channel 2814 by other means. For example, the droplets 2820 can be moved in the channel 2814 using fluidic flow, dielectrophoresis, or the like.

The droplet generator 2806 itself can be part of an EW section (e.g., EW section 124 in the drawings of the present application) of a microfluidic device and can thus comprise an EW configuration with a photoconductive substrate (e.g., as illustrated in U.S. Patent No. 6,958,132), a photo-actuated circuit substrate (e.g., as illustrated in U.S. Patent Application Publication No. 2014/0124370 (attorney docket no. BL9-US)), a phototransistor-based substrate (e.g., as illustrated in U.S. Patent No. 7,956,339), or an electrically-actuated circuit substrate (e.g., as illustrated in U.S. Patent No. 8,685,344). Alternatively, the droplet generator can have a T- or Y-shaped hydrodynamic structure (e.g., as illustrated in U.S. Patents & Patent Application Publication Nos. 7,708,949, 7,041,481 (reissued as RE41,780), 2008/0014589, 2008/0003142, 2010/0137163, and 2010/0172803).

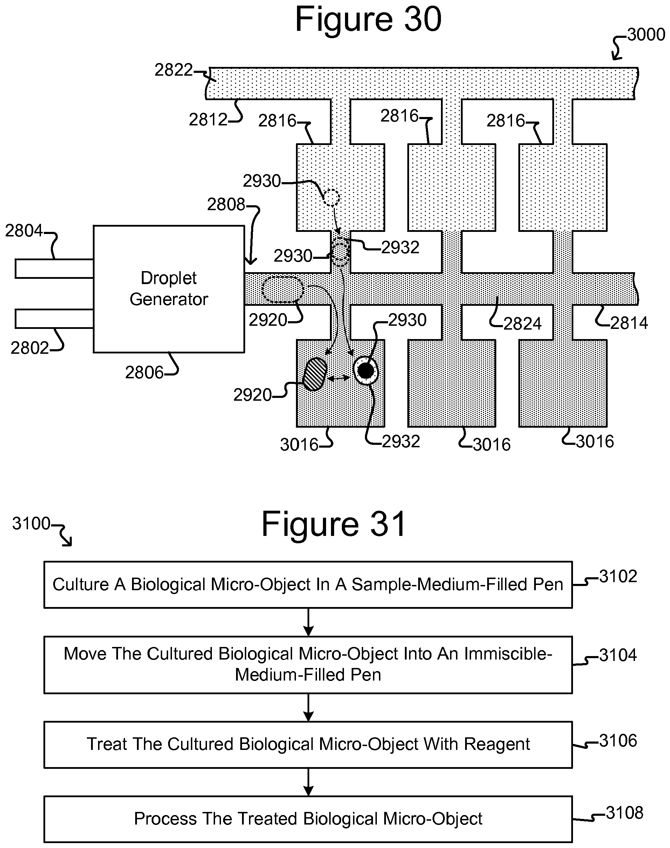

Figures 29 and 30 illustrate examples of alternative microfluidic circuits 2900 and 3000 that include holding pens 2916 and 3016, respectively, which are fluidically connected to the sample channel 2814 but not to the perfusion channel 2812. In such configurations, if the sample channel 2814 is EW configured, the holding pens 2916 and 3016 can also be EW configured. The illustrations of the microfluidic circuits 2800, 2900, and 3000 are examples only, and variations are possible. For example, holding pens 2816 need not be vertically aligned with pens 3016 in the microfluidic circuit 3000 of Figure 30.

The droplet generator 2806 can be utilized to load biological micro-objects and/or facilitate the running of biochemical and/or molecular biological workflows on the microfluidic device. Figures 28-30 illustrate non-limiting examples.

As shown in Figure 28, the droplet generator 2806 can output into the sample channel 2814 a droplet 2820 of sample material 2822 containing a micro-object 2830. The droplet 2820 can then be moved via the sample channel 2814 into one of the holding pens 2816, as shown in Figure 28. Droplets 2820 generated by the droplet generator 2806 that do not contain a micro-object 2830 can be discarded rather than moved into a holding pen 2816.

Figures 29 and 30 illustrate another example in which the droplet generator 2806 generates a droplet 2920 comprising a reagent (or other biological material). The reagent-containing droplet 2920 can be moved through the sample channel 2814 and into one of the holding pens 2916 or 3016 containing the immiscible medium 2824. Prior to or after moving the reagent-containing droplet 2920 into one of the holding pens 2916 or 3016, one or more micro-objects 2930 in one or more droplets 2932 can be moved into the same holding pen 2916 or 3016. The reagent-containing droplet 2920 can then be merged with the droplet 2932 containing the micro-object 2930, allowing the reagents of droplet 2920 to mix and chemically react with the contents of droplet 2932. The one or more micro-object-containing droplets 2932 can be supplied by the droplet generator 2806, as shown in Figure 28, or can be obtained from a holding pen 2816, as shown in Figures 29 and 30. The micro-object 2930 can be a biological micro-object, such as a cell, which has optionally been cultured (e.g., in a holding pen 2816) prior to being moved to the holding pen 2916 or 3016. Alternatively, the micro-object 2930 can be a bead, such as an affinity bead that is capable of binding to molecules of interest in a sample (e.g., cell secretions present in sample material 2822 after the sample material 2822 has been used to culture one or more biological cells). In still other alternatives, the one or more droplets 2932 can contain no micro-objects but only aqueous medium, such as sample material 2822, e.g., that contains cell secretions after the sample material 2822 has been used to culture one or more biological cells.

Figure 31 illustrates an example of a process 3100 that can be performed in a microfluidic device comprising a droplet generator 2806 and microfluidic circuit like any of 2800, 2900, or 3000.

At step 3102 of the process 3100, a biological micro-object can be cultured in a holding pen filled with a sample medium (e.g., cell culture medium). For example, a micro-object 2830 of Figure 28 or a micro-object 2930 in Figures 29 and 30 can be biological and can be cultured in its holding pen. Culturing can be generally as discussed above with respect to step 2204 of Figure 22. For example, culturing can include perfusing the channel 2812 with the sample medium 2822 and/or other culturing media. Step 3102 can be performed over a specified period of time.

At step 3104, the cultured biological micro-object can be moved from the sample-medium-filled holding pen in which it was cultured to a holding pen filled with a medium in which the sample medium is immiscible. For example, the cultured micro-object 2830 or 2930 can be moved in a droplet 2820 or 2932 of sample medium 2822 from one of the holding pens 2816 into one of the holding pens 2916 or 3016, as illustrated in Figure 29 or 30 and discussed above.

At step 3106, the cultured biological micro-object can be subjected to one or more treatments or processes in the immiscible-medium-filled holding pen. For example, one or more droplets 2920 containing one or more reagents can be produced by the droplet generator 2806 and moved into the immiscible-medium-filled holding pen 2916 or 3016 and merged with the droplet 2932 containing the cultured biological micro-object 2830, as shown in Figure 29 or 30 and discussed above. For example, a first reagent-containing droplet 2920 can contain a lysing reagent. Merger of the droplet 3932 containing the cultured biological micro-object 2830 with the first reagent-containing droplet 2920 containing lysing reagent, would result in the lysis of the cultured biological micro-object 2830. In other words, a single new droplet (not shown) would be formed that contains a cell lysate from the cultured biological micro-object 2830. Additional (e.g., second, third, fourth, etc.) reagent-containing droplets 2920 could then be merged with the cell lysate-containing new droplet, so as to further process the cell lysate as desired.

In addition or as another example, one or more droplets containing one or more labeled capture micro-objects (not shown) having an affinity for a secretion or other material or materials of interest (e.g., nucleic acids such as DNA or RNA, proteins, metabolites, or other biological molecules) produced the cultured biological micro-object 2830 can be generated by the droplet generator 2806 and moved into the immiscible-medium-filled pen 2916 or 3016 and merged with the droplet of sample medium 2822 containing the cultured biological micro-object 2830 in a similar manner. In cases where the cultured biological micro-object 2830 has already been lysed, capture micro-object-containing droplet 2920 could contain one or more affinity beads (e.g., having affinity for nucleic acids, such as DNA, RNA, microRNAs, or the like) which, upon merger with the cell lysate-containing droplet in holding pen 2916 or 3016, could bind to target molecules present in the lysate.

At step 3108, the treated biological micro-object can be optionally processed. For example, if at step 3106, a capture object (not shown) is moved into the immiscible-medium-filled pen 2916 or 3016 with the cultured biological micro-object 2830, the pen 2916 or 3016 can be monitored at step 3108 for a reaction (e.g., a fluorescent signal) indicative of a quantity of the material of interest bound to the labeled capture micro-object. Alternatively, such a capture micro-object (not shown) can be removed (e.g., in a droplet 2922) from the pen 2916 or 3016 and exported from the microfluidic device (not shown in Figures 28-30) for subsequent analysis. As yet another example, the treated biological micro-object 2830 can be removed (e.g., in a droplet 2932) from the pen 2916 or 3016 and exported from the microfluidic device (not shown) for subsequent analysis.

Although specific embodiments and applications of the invention have been described in this specification, these embodiments and applications are exemplary only, and many variations are possible. For example, the method of Figure 31 can be performed with respect to sample material contain cell secretions (e.g., after the sample material 2822 has been used to culture one or more biological cells). In such an embodiment, step 3102 would remain the same, but step 3104 would involve moving droplets 2932 which can contain no micro-objects but only aqueous medium, such as sample material 2822 containing cell secretions, into immiscible-medium-containing holding pens 2916 or 3016, and steps 3106 and 3108 would be performed with respect to such aqueous medium-containing droplets 2932. Furthermore, the DEP configurations (e.g., 122) illustrated in the drawings or described herein are examples. Generally speaking, the DEP configurations (e.g., 122) can be any type of optoelectronic tweezers (OET) device known in the art, examples of which are disclosed in U.S. Patent No. 7,612,355 (now RE44,711), U.S. Patent No. 7,956,339, and U.S. Patent Application Publication No.2014/0124370. Other examples of the DEP configurations include any kind of electronically controlled electronic tweezers, an example of which is disclosed in U.S. Patent No. 6,942,776. Generally speaking, the EW configurations can be any type of optoelectronic wetting (OEW) devices known in the art, examples of which are disclosed in U.S. Patent No. 6,958,132. Other examples of EW configurations include electrowetting on dielectric (EWOD) devices, which can be electronically controlled, an example of which is disclosed in U.S. Patent No. 8,685,344.

## Claims

1. An apparatus (1000) comprising:
an enclosure (102) configured to hold a first liquid medium disposed on a first surface (182) in a first section (122) of said enclosure and a second liquid medium disposed on an electrowetting surface (184) in a second section (124) of said enclosure, wherein said enclosure comprises:
a first microfluidic channel (1012);
a second microfluidic channel (1014);
microfluidic holding pens (1016), each holding pen connected to one or both of the first and second channels; and
a boundary (126) between said first section and said second section of said enclosure, said boundary comprising a physical barrier (128) located in said enclosure between said first section of said enclosure and said second section of said enclosure and a passage from said first section of said enclosure through said barrier to said second section of said enclosure;
wherein:
said first section of said enclosure comprises a DEP configuration configured to induce selectively net dielectrophoresis (DEP) forces in said first liquid medium sufficiently to capture and move, relative to said first surface, micro-objects in said first liquid medium in said first section of said enclosure while connected to a biasing device, and
said second section of said enclosure comprises an electrowetting (EW) configuration configured to change selectively an effective wetting characteristic of regions of said electrowetting surface sufficiently to move a liquid droplet within said second medium in said second section of said enclosure while connected to a biasing device.

2. The apparatus of claim 1, wherein said enclosure comprises:
a first biasing electrode (168) disposed on one side of said enclosure,
a dielectric hydrophobic material (160) disposed on an opposite side of said enclosure,
a second biasing electrode (158) disposed on said opposite side of said enclosure, and
an electrode activation substrate (154) disposed between said dielectric hydrophobic material and said second biasing electrode,
wherein optionally said dielectric hydrophobic material is part of said EW configuration but not part of said DEP configuration.

3. The apparatus of claim 2, wherein said electrode activation substrate comprises a photoconductive material.

4. The apparatus of claim 1, wherein said first surface and said electrowetting surface are disposed substantially in a same plane in said enclosure.

5. The apparatus of claim 1, wherein each holding pen is connected to said first channel and said second channel.

6. The apparatus of claim 1, further comprising a droplet generator (2806) configured to selectively provide droplets of one or more media into said second microfluidic channel.

7. The apparatus of claim 1, wherein:
said first section of said enclosure comprises said first channel, and
said second section of said enclosure comprises said second channel.

8. The apparatus of claim 7, wherein said first section of said enclosure further comprises said holding pens.

9. A process of operating a fluidic apparatus having an enclosure that comprises a first surface and an electrowetting surface, wherein said fluidic apparatus is an apparatus of any of the preceding claims, said process comprising:
drawing (1106) a droplet of a first liquid medium disposed on said first surface in a first section of said enclosure into a second medium disposed on said electrowetting surface in a second section of said enclosure,
wherein said drawing comprises changing (2704) an effective electrowetting characteristic of a region of said electrowetting surface at a boundary with said first surface to induce a sufficient force at said region on said droplet to draw said droplet across said boundary and into said second liquid medium,
wherein said region of said electrowetting surface is adjacent a passage through a physical barrier at said boundary, and said changing comprises drawing said droplet of said first medium through said passage into said second medium,
wherein said first medium is an aqueous medium and said second medium is a medium that is immiscible in said aqueous medium, and
wherein said droplet contains a micro-object.

10. The process of claim 9 further comprising:
selecting said micro-object from a plurality of micro-objects in said first liquid medium, and
moving said selected micro-object in said first liquid medium to said boundary adjacent said region of said electrowetting surface,
wherein said selecting comprises activating dielectrophoresis (DEP) electrodes at said first surface of said enclosure to create a net DEP force sufficient to capture said selected micro-object,
wherein said moving comprises further activating and deactivating DEP electrodes at said first surface to move said selected micro-object to said boundary adjacent said region of said electrowetting surface, and
wherein said activating and deactivating said DEP electrodes at said first surface of said enclosure optionally comprises directing a changing pattern of light onto said first surface of said enclosure.

11. The process of claim 9, wherein said changing comprises activating EW electrodes at said region of said electrowetting surface, and wherein said activating said EW electrodes at said region of said electrowetting surface optionally comprises directing a pattern of light onto said region of said electrowetting surface.

12. The process of claim 9, wherein said first surface of said enclosure and said electrowetting surface are located substantially in a same plane.

13. The process of claim 9, wherein:
said first section of said enclosure comprises the first microfluidic channel and the microfluidic holding pens disposed on said first surface of said enclosure,
said second section of said enclosure comprises the second microfluidic channel disposed on said electrowetting surface of said enclosure, and
said process further comprises culturing biological micro-objects in said holding pens.

14. The process of claim 13, wherein:
said droplet comprises an aliquot of said first medium in one of said holding pens, said aliquot comprising biological material from one of said biological micro-objects in said one of said holding pens, and
said drawing comprises drawing said droplet from said one of said pens into said second channel.

15. The process of claim 13, further comprising moving said one of said biological micro-objects in said one of said holding pens to said boundary adjacent said region of said electrowetting surface, wherein:
said droplet comprises one of said biological micro-objects from one of said holding pens, and
said drawing comprises drawing said droplet from said one of said holding pens into said second channel.

16. The process of claim 13 further comprising:
moving said biological micro-objects from said first medium in said first channel into said holding pens, and
replacing said first medium in said first channel with said second medium.

17. The process of claim 9, wherein said drawing further comprises inducing a pressure differential between said first liquid medium and said second liquid medium to draw said droplet across said boundary and into said second liquid medium.

## Patentansprüche

1. Vorrichtung (1000), umfassend:
ein Gehäuse (102), das dafür ausgelegt ist, ein erstes flüssiges Medium, das auf einer ersten Oberfläche (182) in einem ersten Abschnitt (122) des Gehäuses angeordnet ist, und ein zweites flüssiges Medium, das auf einer Elektrobenetzungsoberfläche (184) in einem zweiten Abschnitt (124) des Gehäuses angeordnet ist, aufzunehmen, wobei das Gehäuse umfasst:
einen ersten mikrofluidischen Kanal (1012);
einen zweiten mikrofluidischen Kanal (1014);
mikrofluidische Buchten (1016), wobei jede Bucht mit dem ersten und/oder dem zweiten Kanal verbunden ist; und
eine Grenze (126) zwischen dem ersten Abschnitt und dem zweiten Abschnitt des Gehäuses, wobei die Grenze eine physische Barriere (128), die sich im Gehäuse zwischen dem ersten Abschnitt des Gehäuses und dem zweiten Abschnitt des Gehäuses befindet, und eine Durchführung vom ersten Abschnitt des Gehäuses durch die Barriere zum zweiten Abschnitt des Gehäuses umfasst;
wobei:
der erste Abschnitt des Gehäuses eine DEP-Konfiguration umfasst, die dafür ausgelegt ist, ausreichend Netto-Dielektrophorese (DEP)-Kräfte selektiv im ersten flüssigen Medium zu induzieren, um Mikroobjekte im ersten flüssigen Medium im ersten Abschnitt des Gehäuses einzufangen und relativ zur ersten Oberfläche zu bewegen, während eine Verbindung mit einer Vorspannungsvorrichtung besteht, und
der zweite Abschnitt des Gehäuses eine Elektrobenetzungs (Electrowetting, EW)-Konfiguration umfasst, die dafür ausgelegt ist, selektiv eine effektive Benetzungseigenschaft von Bereichen der Elektrobenetzungsoberfläche ausreichend zu ändern, um einen flüssigen Tropfen im zweiten Medium im zweiten Abschnitt des Gehäuses zu bewegen, während eine Verbindung mit einer Vorspannungsvorrichtung besteht.

2. Vorrichtung nach Anspruch 1, wobei das Gehäuse umfasst:
eine erste Vorspannungselektrode (168), die auf einer Seite des Gehäuses angeordnet ist,
ein dielektrisches hydrophobes Material (160), das auf einer gegenüberliegenden Seite des Gehäuses angeordnet ist,
eine zweite Vorspannungselektrode (158), die auf der gegenüberliegenden Seite des Gehäuses angeordnet ist, und
ein Elektrodenaktivierungssubstrat (154), das zwischen dem dielektrischen hydrophoben Material und der zweiten Vorspannungselektrode angeordnet ist,
wobei wahlweise das dielektrische hydrophobe Material Teil der EW-Konfiguration, jedoch nicht Teil der DEP-Konfiguration ist.

3. Vorrichtung nach Anspruch 2, wobei das Elektrodenaktivierungssubstrat ein photoleitfähiges Material umfasst.

4. Vorrichtung nach Anspruch 1, wobei die erste Oberfläche und die Elektrobenetzungsoberfläche im Wesentlichen in derselben Ebene im Gehäuse angeordnet sind.

5. Vorrichtung nach Anspruch 1, wobei jede Bucht mit dem ersten Kanal und dem zweiten Kanal verbunden ist.

6. Vorrichtung nach Anspruch 1, ferner ein Tropfenerzeugungselement (2806) umfassend, das dafür ausgelegt ist, Tropfen eines oder mehrerer Medien selektiv in den zweiten mikrofluidischen Kanal abzugeben.

7. Vorrichtung nach Anspruch 1, wobei:
der erste Abschnitt des Gehäuses den ersten Kanal umfasst, und
der zweite Abschnitt des Gehäuses den zweiten Kanal umfasst.

8. Vorrichtung nach Anspruch 7, wobei der erste Abschnitt des Gehäuses ferner die Buchten umfasst.

9. Prozess des Betreibens einer fluidischen Vorrichtung mit einem Gehäuse, das eine erste Oberfläche und eine Elektrobenetzungsoberfläche umfasst, wobei die fluidische Vorrichtung eine Vorrichtung nach einem der vorstehenden Ansprüche ist, wobei der Prozess umfasst:
Ziehen (1106) eines Tropfens eines ersten flüssigen Mediums, das auf der ersten Oberfläche in einem ersten Abschnitt des Gehäuses angeordnet ist, in ein zweites Medium, das auf der Elektrobenetzungsoberfläche in einem zweiten Abschnitt des Gehäuses angeordnet ist,
wobei das Ziehen das Ändern (2704) einer effektiven Elektrobenetzungseigenschaft eines Bereichs der Elektrobenetzungsoberfläche an einer Grenze zur ersten Oberfläche umfasst, um eine ausreichende Kraft in dem Bereich auf den Tropfen zu induzieren, um den Tropfen über die Grenze und in das zweite flüssige Medium zu ziehen,
wobei der Bereich der Elektrobenetzungsoberfläche an eine Durchführung durch eine physische Barriere an der Grenze angrenzt und das Ändern das Ziehen des Tropfens des ersten Mediums durch die Durchführung in das zweite Medium umfasst,
wobei das erste Medium ein wässriges Medium ist und das zweite Medium ein Medium ist, das mit dem wässrigen Medium nicht mischbar ist, und
wobei der Tropfen ein Mikroobjekt enthält.

10. Prozess nach Anspruch 9, ferner umfassend:
Auswählen des Mikroobjekts aus mehreren Mikroobjekten im ersten flüssigen Medium, und
Bewegen des ausgewählten Mikroobjekts im ersten flüssigen Medium zu der Grenze, die an den Bereich der Elektrobenetzungsoberfläche angrenzt,
wobei das Auswählen das Aktivieren von Dielektrophorese (DEP)-Elektroden an der ersten Oberfläche des Gehäuses umfasst, um eine Netto-DEP-Kraft zu erzeugen, die ausreicht, um das ausgewählte Mikroobjekt einzufangen,
wobei das Bewegen ferner das Aktivieren und Deaktivieren von DEP-Elektroden an der ersten Oberfläche umfasst, um das ausgewählte Mikroobjekt zu der Grenze zu bewegen, die an den Bereich der Elektrobenetzungsoberfläche angrenzt, und
wobei das Aktivieren und Deaktivieren der DEP-Elektroden an der ersten Oberfläche des Gehäuses wahlweise das Leiten eines sich ändernden Lichtmusters auf die erste Oberfläche des Gehäuses umfasst.

11. Prozess nach Anspruch 9, wobei das Ändern das Aktivieren von EW-Elektroden im Bereich der Elektrobenetzungsoberfläche umfasst und wobei das Aktivieren der EW-Elektroden im Bereich der Elektrobenetzungsoberfläche wahlweise das Leiten eines Lichtmusters auf den Bereich der Elektrobenetzungsoberfläche umfasst.

12. Prozess nach Anspruch 9, wobei sich die erste Oberfläche des Gehäuses und die Elektrobenetzungsoberfläche im Wesentlichen in derselben Ebene befinden.

13. Prozess nach Anspruch 9, wobei:
der erste Abschnitt des Gehäuses den ersten mikrofluidischen Kanal und die mikrofluidischen Buchten umfasst, die auf der ersten Oberfläche des Gehäuses angeordnet sind,
der zweite Abschnitt des Gehäuses den zweiten mikrofluidischen Kanal umfasst, der auf der Elektrobenetzungsoberfläche des Gehäuses angeordnet ist, und
der Prozess ferner das Kultivieren von biologischen Mikroobjekten in den Buchten umfasst.

14. Prozess nach Anspruch 13, wobei:
der Tropfen ein Aliquot des ersten Mediums in einer der Buchten umfasst, wobei das Aliquot biologisches Material von einem der biologischen Mikroobjekte in der einen der Buchten umfasst, und
das Ziehen das Ziehen des Tropfens aus der einen der Buchten in den zweiten Kanal umfasst.

15. Prozess nach Anspruch 13, ferner umfassend das Bewegen des einen der biologischen Mikroobjekte in der einen der Buchten zu der Grenze, die an den Bereich der Elektrobenetzungsoberfläche angrenzt, wobei:
der Tropfen eins der biologischen Mikroobjekte aus einer der Buchten umfasst, und
das Ziehen das Ziehen des Tropfens aus der einen der Buchten in den zweiten Kanal umfasst.

16. Prozess nach Anspruch 13, ferner umfassend:
Bewegen der biologischen Mikroobjekte aus dem ersten Medium im ersten Kanal in die Buchten, und
Ersetzen des ersten Mediums im ersten Kanal durch das zweite Medium.

17. Prozess nach Anspruch 9, wobei das Ziehen ferner das Induzieren eines Druckunterschieds zwischen dem ersten flüssigen Medium und dem zweiten flüssigen Medium umfasst, um den Tropfen über die Grenze und in das zweite flüssige Medium zu ziehen.

## Revendications

1. Appareil (1000) comprenant :
une enceinte (102) conçue pour contenir un premier milieu liquide disposé sur une première surface (182) dans une première section (122) de ladite enceinte et un second milieu liquide disposé sur une surface d'électromouillage (184) dans une seconde section (124) de ladite enceinte, dans lequel ladite enceinte comprend :
un premier canal microfluidique (1012) ;
un second canal microfluidique (1014) ;
des zones de retenue microfluidique (1016), chaque zone de retenue étant reliée à un des premier et second canaux ou aux deux ; et
une frontière (126) entre ladite première section et ladite seconde section de ladite enceinte, ladite frontière comprenant une barrière physique (128) située dans ladite enceinte entre ladite première section de ladite enceinte et ladite seconde section de ladite enceinte et un passage depuis ladite première section de ladite enceinte à travers ladite barrière vers ladite seconde section de ladite enceinte ;
dans lequel :
ladite première section de ladite enceinte comprend une configuration DEP conçue pour induire des forces de diélectrophorèse (DEP) nettes sélectivement dans ledit premier milieu liquide suffisamment pour capturer et déplacer, par rapport à ladite première surface, des micro-objets dans ledit premier milieu liquide dans ladite première section de ladite enceinte tout en étant reliée à un dispositif de polarisation, et
ladite seconde section de ladite enceinte comprend une configuration d'électromouillage (EW) conçue pour changer sélectivement une caractéristique d'électromouillage efficace de régions de ladite surface d'électromouillage suffisamment pour déplacer une gouttelette de liquide dans ledit second milieu dans ladite seconde section de ladite enceinte tout en étant reliée à un dispositif de polarisation.

2. Appareil selon la revendication 1, dans lequel ladite enceinte comprend :
une première électrode de polarisation (168) disposée sur un côté de ladite enceinte,
un matériau diélectrique hydrophobe (160) disposé sur un côté opposé de ladite enceinte,
une seconde électrode de polarisation (158) disposée sur ledit côté opposé de ladite enceinte, et
un substrat d'activation d'électrode (154) disposé entre ledit matériau diélectrique hydrophobe et ladite seconde électrode de polarisation,
dans lequel éventuellement ledit matériau diélectrique hydrophobe fait partie de ladite configuration EW, mais ne fait pas partie de ladite configuration DEP.

3. Appareil selon la revendication 2, dans lequel ledit substrat d'activation d'électrode comprend un matériau photoconducteur.

4. Appareil selon la revendication 1, dans lequel ladite première surface et ladite surface d'électromouillage sont disposées sensiblement dans un même plan dans ladite enceinte.

5. Appareil selon la revendication 1, dans lequel chaque zone de retenue est reliée audit premier canal et audit second canal.

6. Appareil selon la revendication 1, comprenant en outre un générateur (2806) de gouttelettes conçu pour fournir sélectivement des gouttelettes d'un ou de plusieurs milieux dans ledit second canal microfluidique.

7. Appareil selon la revendication 1, dans lequel :
ladite première section de ladite enceinte comprend ledit premier canal, et
ladite seconde section de ladite enceinte comprend ledit second canal.

8. Appareil la revendication 7, dans lequel ladite première section de ladite enceinte comprend en outre lesdites zones de retenue.

9. Procédé d'utilisation d'un appareil fluidique possédant une enceinte qui comprend une première surface et une surface d'électromouillage, dans lequel ledit appareil fluidique est un appareil selon l'une quelconque des revendications précédentes, ledit procédé consistant à :
aspirer (1106) une gouttelette d'un premier milieu liquide disposé sur ladite première surface dans une première section de ladite enceinte vers un second milieu disposé sur ladite surface d'électromouillage dans une seconde section de ladite enceinte,
dans lequel ladite aspiration consiste à changer (2704) une caractéristique d'électromouillage efficace d'une région de ladite surface d'électromouillage au niveau d'une frontière avec ladite première surface pour induire une force suffisante au niveau de ladite région sur ladite gouttelette pour aspirer ladite gouttelette à travers ladite frontière et vers ledit second milieu liquide,
dans lequel ladite région de ladite surface d'électromouillage est adjacente à un passage à travers une barrière physique au niveau de ladite frontière, et ledit changement consiste à aspirer ladite gouttelette dudit premier milieu à travers ledit passage vers ledit second milieu,
dans lequel ledit premier milieu est un milieu aqueux et ledit second milieu est un milieu qui n'est pas miscible dans ledit milieu aqueux, et
dans lequel ladite gouttelette contient un micro-objet.

10. Procédé selon la revendication 9 consistant en outre à :
sélectionner ledit micro-objet dans une pluralité de micro-objets dans ledit premier milieu liquide, et
déplacer ledit micro-objet sélectionné dans ledit premier milieu liquide vers ladite frontière adjacente à ladite région de ladite surface d'électromouillage,
dans lequel ladite sélection consiste à activer des électrodes de diélectrophorèse (DEP) au niveau de ladite première surface de ladite enceinte afin de créer une force DEP nette suffisante pour capturer ledit micro-objet sélectionné,
dans lequel ledit déplacement consiste en outre à activer et désactiver les électrodes DEP au niveau de ladite première surface pour déplacer ledit micro-objet sélectionné vers ladite frontière adjacente à ladite région de ladite surface d'électromouillage, et
dans lequel ladite activation et ladite désactivation desdites électrodes DEP au niveau de ladite première surface de ladite enceinte consistent éventuellement à diriger un changement de motif de lumière sur ladite première surface de ladite enceinte.

11. Procédé selon la revendication 9, dans lequel ledit changement consiste à activer les électrodes EW au niveau de ladite région de ladite surface d'électromouillage, et dans lequel ladite activation desdites électrodes EW au niveau de ladite région de ladite surface d'électromouillage consiste éventuellement à orienter un motif de lumière sur ladite région de ladite surface d'électromouillage.

12. Procédé selon la revendication 9, dans lequel ladite première surface de ladite enceinte et ladite surface d'électromouillage sont situées sensiblement dans un même plan.

13. Procédé selon la revendication 9, dans lequel :
ladite première section de ladite enceinte comprend le premier canal microfluidique et les zones de retenue microfluidique disposées sur ladite première surface de ladite enceinte,
ladite seconde section de ladite enceinte comprend le second canal microfluidique disposé sur ladite surface d'électromouillage de ladite enceinte, et
ledit procédé consiste en outre à mettre en culture des micro-objets biologiques dans lesdites zones de retenue.

14. Procédé selon la revendication 13, dans lequel :
ladite gouttelette comprend une aliquote dudit premier milieu dans l'une des zones de retenue, ladite aliquote comprenant une substance biologique provenant de l'un desdits micro-objets biologiques dans ladite une desdites zones de retenue, et
ladite aspiration consiste à aspirer ladite gouttelette depuis ladite une desdites zones vers ledit second canal.

15. Procédé selon la revendication 13, consistant en outre à déplacer ledit un desdits micro-objets biologiques dans ladite une desdites zones de retenue vers ladite frontière adjacente à ladite région de ladite surface d'électromouillage, dans lequel :
ladite gouttelette comprend un desdits micro-objets biologiques provenant de l'une desdites zones de retenue, et
ladite aspiration consiste à aspirer ladite gouttelette depuis ladite une desdites zones de retenue vers ledit second canal.

16. Procédé selon la revendication 13 consistant en outre à :
déplacer lesdits micro-objets biologiques depuis ledit premier milieu dans ledit premier canal vers lesdites zones de retenue, et
remplacer ledit premier milieu dans ledit premier canal par ledit second milieu.

17. Procédé selon la revendication 9, dans lequel ladite aspiration consiste en outre à induire un différentiel de pression entre ledit premier milieu liquide et ledit second milieu liquide pour aspirer ladite gouttelette à travers ladite frontière et vers ledit second milieu liquide.
